(19) Europäisches Patentamt
European Patent Office
Office européen des brevets

(11) **EP 3 442 491 B1**

(12) **EUROPÄISCHE PATENTSCHRIFT**

(45) Veröffentlichungstag und Bekanntmachung des Hinweises auf die Patenterteilung:
**24.02.2021 Patentblatt 2021/08**

(21) Anmeldenummer: **17716285.6**

(22) Anmeldetag: **13.04.2017**

(51) Int Cl.:
*A61K 8/26* (2006.01)     *C09C 1/64* (2006.01)
*C09C 1/00* (2006.01)     *C09C 1/62* (2006.01)
*C09C 1/66* (2006.01)

(86) Internationale Anmeldenummer:
**PCT/EP2017/058980**

(87) Internationale Veröffentlichungsnummer:
**WO 2017/178610 (19.10.2017 Gazette 2017/42)**

(54) **OBERFLÄCHENMODIFIZIERTES EFFEKTPIGMENT UND NAGELLACKKOMPOSITION**

SURFACE MODIFIED EFFECT PIGMENT AND NAIL VARNISH COMPOSITION

PIGMENT A EFFET A SURFACE MODIFIE ET COMPOSITION DE VERNIS A ONGLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2016 EP 16000855**

(43) Veröffentlichungstag der Anmeldung:
**20.02.2019 Patentblatt 2019/08**

(60) Teilanmeldung:
**20176997.3 / 3 738 577**

(73) Patentinhaber: **ECKART GmbH**
**91235 Hartenstein (DE)**

(72) Erfinder:
• **GEBHARD, Ann-Katrin**
**91235 Hartenstein (DE)**
• **PIPPINGER, Christina**
**91235 Hartenstein (DE)**
• **SCHMIDT, Ulrich**
**91235 Hartenstein (DE)**
• **SCHILLING, Christine**
**91235 Hartenstein (DE)**

(74) Vertreter: **Altana IP Department**
**Altana Management Services GmbH**
**Abelstraße 45**
**46483 Wesel (DE)**

(56) Entgegenhaltungen:
DE-A1- 19 820 112     DE-A1-102005 036 333
DE-A1-102007 034 928     US-A1- 2010 047 199

EP 3 442 491 B1

**Beschreibung**

Die vorliegende Erfindung betrifft ein oberflächenmodifiziertes Effektpigment sowie ein Verfahren zur Herstellung desselben sowie Nagellackkompositionen enthaltend dieses oberflächenmodifizierte Effektpigment.

EP 1 812 518 A2 offenbart Perlglanzpigmente, welche an der Oberfläche mit wenigstens einer phosphorhaltigen Verbindung versehen sind. Die Perlglanzpigmente eignen sich insbesondere für die Verwendung in Pulverlacken.

EP 2 227 508 A1 beschreibt mit mindestens einer Metalloxidschicht beschichtete Metalleffektpigmente, wobei die Oberfläche der Metalloxidschicht mindestens ein fluoralkyl- und/oder fluorarylhaltiges Oberflächenmodifizierungsmittel bzw. kovalent gebundenes Polysiloxan aufweist. Die Metalleffektpigmente kommen insbesondere in Pulverlacken zu Anwendung.

EP 2 318 463 A1 offenbart mit mindestens einer Metalloxidschicht beschichtete Metalleffektpigmente, wobei die Oberfläche der Metalloxidschicht kovalent gebundenes Polysiloxan aufweist. Die Metalleffektpigmente eignen sich insbesondere zur Verwendung in Pulverlacken.

EP 2 576 702 A1 betrifft die Verwendung oberflächenmodifizierter Effektpigmente insbesondere in Pulverlacken. Die Effektpigmentoberfläche ist hier mit wenigstens einer epoxidgruppenhaltigen Verbindung oberflächenmodifiziert.

EP 1 462 085 A1 offenbart eine Nagellackkomposition mit Spiegeleffekt, welche Partikel mit metallischem Glanz in einem Anteil von ≥ 2 Gew.-%, bezogen auf das Gesamtgewicht der Nagellackkomposition, umfasst. EP 1 462 085 A1 offenbart keine oberflächenmodifizierten Effektpigmente.

EP 1 299 066 A2 beschreibt einen Aluminiumplättchen enthaltenden Nagellack mit spiegelartigem Aussehen. Gemäß EP 1 299 066 A2 muss der Nagellack als Filmbildner Nitrocellulose mit einem Molekulargewicht von > 56000 umfassen, damit ein spiegelartiger Effekt auf einem Fingernagel erzielt werden kann. Dennoch sind die hiermit erreichbaren Spiegeleffekte limitiert und verbesserungswürdig.

EP 1 746 913 A2 offenbart einen flexiblen Artikel umfassend wenigstens eine Klebeschicht zur Fixierung des Artikels auf einem Fingernagel, wenigstens einen organischen Film sowie wenigstens eine, für z.B. einen optischen Effekt verantwortliche Komponente. Letztere kann beispielsweise dazu dienen, dem flexiblen Artikel einen Spiegeleffekt zu verleihen.

EP 1 792 598 A1 beschreibt einen Nagellack mit Spiegelglanz, welcher kolloidale Edelmetallpartikel mit einer mittleren Partikelgröße von 10 bis 100 nm in einem Anteil von 5 bis 50 Gew.-%, bezogen auf das Gesamtgewicht des Nagellacks, enthält.

EP 1 796 794 A1 offenbart eine kosmetische Zusammensetzung, welche ein PVD-Aluminiumpigment in einer Pigmentierungshöhe von 0,05 bis 5,0 Gew.-%, bezogen auf das Gesamtgewicht der kosmetischen Zusammensetzung, und wenigstens ein leafing-Additiv umfasst. Als leafing-Additiv dienen langkettige Phosphorsäureester oder ein Gemisch aus mehreren langkettigen Phosphorsäureestern. In den meisten Nagellacken jedoch kommt der leafing-Effekt nicht voll zur Geltung.

EP 1 082 952 A1 offenbart eine kosmetische Zusammensetzung, beispielsweise einen Nagellack, welche metallbeschichtete Glaspartikel umfasst.

JP 2012081236 A offenbart die Befestigung eines Spiegels an einem Fingernagel.

EP 2 248 514 A2 beschreibt nitrocellulosefreie Nagellackkompositionen, welche wenigstens ein Styrol/Maleinsäureanhydrid-Copolymer als hochglänzenden Filmbildner, wenigstens ein Epoxyharz als Co-Filmbildner, wenigstens eine reaktive Komponente sowie wenigstens ein Lösungsmittel umfasst. Die nitrocellulosefreie Nagellackkomposition soll vergleichbare oder bessere Haftungseigenschaften als nitrocellulosehaltige Nagellackkompositionen aufweisen. In EP 2 248 514 A2 kommen keine oberflächenmodifizierten Effektpigmente als Farbmittel zum Einsatz.

US 2010/047199 A1 offenbart sehr dünne, naßvermahlene Aluminiumeffektpigmente mit enger Dickenverteilung. Die DE 10 2007 034928 A1 offenbart spezielle dreischichtige Effektpigmente, die mittels einer reaktiven Verdampfung von Metallen im PVD-Verfahren hergestellt werden. Beide Dokumente offenbaren jedoch keinen geeigneten Nagellack, in dem diese Effektpigmente in leafing-Form verwendet werden können.

Aufgabe der vorliegenden Erfindung ist es, ein Effektpigment für die Verwendung in einer Nagellackkomposition bereitzustellen, welches das optische Erscheinungsbild der Nagellackkomposition nach Applikation und Trocknung maßgeblich bestimmt. Im Fall von hochwertigen Metalleffektpigmenten soll ein Spiegelglanz und bevorzugt ein besonderer Spiegelglanz ermöglicht werden.

Insbesondere ist es die Aufgabe der vorliegenden Erfindung, eine Nagellackkomposition bereitzustellen, die einen guten leafing Effekt oberflächenmodifizierter Effektpigmente ermöglicht.

Die der Erfindung zugrunde liegende Aufgabe beinhaltet auch die Bereitstellung eines oberflächenmodifizierten Effektpigments umfassend ein optional mit wenigstens einem Metalloxid, Metallhydroxid und/oder Metalloxidhydrat beschichtetes, metallisches plättchenförmiges Substrat oder ein optional mit wenigstens einem Metalloxid, Metallhydroxid und/oder Metalloxidhydrat beschichtetes nichtmetallisches plättchenförmiges Substrat, wobei

a) das metallische plättchenförmige Substrat durch Nassvermahlung hergestellt wird und einen $h_{50}$-Wert aus einem

Bereich von 20 nm bis kleiner 100 nm aufweist und als Ausgangsmaterial (Additiv) zur Oberflächenmodifizierung

i) Phosphorsäurecetylester oder Cetylphosphorsäure aus einem Bereich von 2 Gew.-% bis unter 40 Gew.-% oder
ii) Phosphorsäurestearylester oder Stearylphosphat aus einem Bereich von 5 Gew.-% bis unter 20 Gew.-% oder
iii) Phosphonsäuren der Formel $R-P(O)(OH)_2$ mit R = linearer Alkylrest mit einer Kohlenstoffkette aus einem Bereich von $C_8$ bis $C_{14}$, in einer Gesamtmenge bevorzugt aus einem Bereich von 4 Gew.-% bis 45 Gew.-% jeweils bezogen auf das Gesamtgewicht des optional beschichteten, metallischen plättchenförmigen Substrats verwendet wird oder

b) das metallische plättchenförmige Substrat durch PVD-Verfahren hergestellt wird und eine mittlere Dicke $h_{50}$ aus einem Bereich von 13 nm bis 60 nm aufweist und als Ausgangsmaterial (Additiv) zur Oberflächenmodifizierung

i) Phosphorsäurecetylester oder Cetylphosphorsäure aus einem Bereich von 10 Gew.-% bis 50 Gew.-% oder
ii) Phosphorsäurestearylester oder Stearylphosphat aus einem Bereich von 13 Gew.-% bis 50 Gew.-% oder
iii) Phosphonsäuren der Formel $R-P(O)(OH)_2$ mit R = linearer Alkylrest mit einer Kohlenstoffkette aus einem Bereich von $C_8$ bis $C_{14}$, in einer Gesamtmenge aus einem Bereich von 5 Gew.-% bis 50 Gew.-%, jeweils bezogen auf das Gesamtgewicht des optional beschichteten, metallischen plättchenförmigen Substrats, verwendet wird oder

c) das nichtmetallische plättchenförmige Substrat einen Dso-Wert aus einem Bereich von 2 $\mu$m bis 360 $\mu$m aufweist und als Ausgangsmaterial (Additiv) zur Oberflächenmodifizierung

i) Phosphorsäurecetylester oder Cetylphosphorsäure aus einem Bereich von 5 Gew.-% bis 30 Gew.-% oder
ii) Phosphorsäurestearylester oder Stearylphosphat aus einem Bereich von 2 Gew.-% bis 25 Gew.-%,

jeweils bezogen auf das Gesamtgewicht des optional beschichteten, nichtmetallischen plättchenförmigen Substrats, verwendet wird oder
d) das nichtmetallische Substrat ein Glasplättchen ist, welches mit metallischen Silber beschichtet ist und einen Dso-Wert aus einem Bereich von 2 $\mu$m bis 360 $\mu$m sowie eine mittlere Dicke $h_{50,Glas}$ des Glasplättchens in einem Bereich von 70 nm bis 530 nm sowie eine mittlere Dicke der metallischen Silberbeschichtung in einem Bereich von 9 nm bis 27 nm aufweist und als Ausgangsmaterial (Additiv) zur Oberflächenmodifizierung

i) Phosphorsäurecetylester oder Cetylphosphorsäure aus einem Bereich von 3 Gew.-% bis unter 40 Gew.-% oder
ii) Phosphorsäurestearylester oder Stearylphosphat aus einem Bereich von 5 Gew.-% bis unter 30 Gew.-% oder
iii) Phosphonsäuren $R-P(O)(OH)_2$ mit R = linearer Alkylrest mit einer Kohlenstoffkette aus einem Bereich von $C_8$ bis $C_{14}$ in einem Anteil aus einem Bereich von 15 Gew.-% bis 43 Gew.-%, jeweils bezogen auf das Gesamtgewicht des mit Silber beschichteten Glasplättchens, verwendet wird.

Die Aufgabe der zugrunde liegende Erfindung wird gelöst durch die Bereitstellung einer Nagellackkomposition enthaltend

a) wenigstens ein mit einem Ausgangsmaterial (Additiv) oberflächenmodifiziertes Effektpigment wobei das Effektpigment ein plättchenförmiges Substrat und optional wenigstens eine auf dem Substrat aufgebrachte Beschichtung umfasst
b) wenigstens ein kohlenwasserstoffhaltiges Harz und
c) wenigstens ein Lösemittel.oder Lösemittelgemisch,

wobei als Ausgangsmaterial (Additiv) zur Oberflächenmodifizierung des Effektpigments wenigstens eine Verbindung aus der Gruppe bestehend aus phosphorsäureesterhaltigen, phosphonsäureesterhaltigen, phosphonsäurehaltigen, fettsäurehaltigen und/oder silanhaltigen Verbindungen oder Mischungen hiervon, entnommen wird.
Bevorzugte Weiterbildungen der Nagellackkomposition sind in den Ansprüchen 2 bis 13 angegeben.
Weiterhin wird die Aufgabe gelöst durch Bereitstellung eines Verfahrens zur Herstellung der erfindungsgemäßen Nagellackkomposition gemäß Anspruch 14.
Die erfindungsgemäß oberflächenmodifizierten Effektpigmente können ein optional mit wenigstens einem Metalloxid, Metallhydroxid und/oder Metalloxidhydrat beschichtetes, metallisches plättchenförmiges Substrat oder ein optional mit wenigstens einem Metalloxid, Metallhydroxid und/oder Metalloxidhydrat beschichtetes nichtmetallisches plättchenförmiges Substrat umfassen.
Die metallischen plättchenförmigen Substrate können über konventionelle Nass- oder Trockenvermahlung oder über PVD-Verfahren hergestellt werden.

Die metallischen plättchenförmigen Substrate können aus der Gruppe, bestehend aus Aluminiumplättchen, Kupferplättchen, Zinkplättchen, Eisenplättchen, Titanplättchen, Edelstahlplättchen, Silberplättchen, Legierungen und Mischungen der vorstehend aufgeführten Metalle, ausgewählt werden. Bevorzugt werden die metallischen plättchenförmigen Substrate aus der Gruppe, bestehend aus Aluminiumplättchen, Kupferplättchen, Zinkplättchen, Eisenplättchen, Edelstahlplättchen, Legierungen und Mischungen der vorstehend aufgeführten Metalle, ausgewählt. Die vorgenannten metallischen plättchenförmigen Substrate können auch eine oder mehrere Schichten aus oder mit wenigstens einem hoch- und/oder niedrigbrechendem Metalloxid, Metallhydroxid und/oder Metalloxidhydrat aufweisen und optional getrocknet und/oder optional kalziniert sein. So können als metallische plättchenförmige Substrate also auch kommerziell erhältliche beschichtete Metalleffektpigmente verwendet werden. Gemäß einer bevorzugten Ausführungsform sind die erfindungsgemäß zu verwendenden metallischen, plättchenförmigen Substrate nicht beschichtet.

Besonders bevorzugt werden die metallischen plättchenförmigen Substrate aus der Gruppe, bestehend aus Aluminiumplättchen, Kupferplättchen, Zinkplättchen, Eisenplättchen, Legierungen und Mischungen der vorstehend aufgeführten Metalle, ausgewählt. Ganz besonders bevorzugt werden die metallischen plättchenförmigen Substrate aus der Gruppe, bestehend aus Aluminiumplättchen, Kupferplättchen, Zinkplättchen, Legierungen und Mischungen der vorstehend aufgeführten Metalle, ausgewählt. Insbesondere bevorzugt werden als metallische plättchenförmige Substrate Aluminiumplättchen eingesetzt.

Weiterhin bevorzugt sind Aluminiumplättchen, die durch PVD-Verfahren hergestellt werden.

Gemäß einer bevorzugten Ausführungsform sind die metallischen plättchenförmigen Substrate PVD-Pigmente, insbesondere Aluminium-PVD-Pigmente.

Diese weisen bevorzugt eine mittlere Dicke $h_{50}$ aus einem Bereich von 13 nm bis 80 nm, weiter bevorzugt aus einem Bereich von 13 nm bis 60 nm und besonders bevorzugt aus einem Bereich von 20 nm bis 40 nm auf.

Unterhalb von 13 nm werden die metallischen PVD-Pigmente, insbesondere Aluminiumpigmente zu dunkel und zu transparent.

Oberhalb von 80 nm lassen die optischen Eigenschaften sowie die Deckkraft erheblich nach und ein Spiegelglanz ist nur schwer erreichbar.

Werden Metalleffektpigmente in kosmetischen Formulierungen eingesetzt, so müssen sie gewissen Reinheitsanforderungen genügen wie beispielsweise die EU Cosmetic Regulation 1223/2009 oder FDA 21CFR part 73.

Werden beispielsweise Aluminiumplättchen als metallisches plättchenförmiges Substrat eingesetzt, weisen diese bevorzugt einen Aluminiumgehalt von ≥ 97 Gew.-%, weiter bevorzugt von ≥ 98 Gew.-%, besonders bevorzugt von ≥ 99 Gew.-% und ganz besonders bevorzugt von ≥ 99,7 Gew.-%, jeweils bezogen auf das Gesamtgewicht des Aluminiumplättchens, auf. Bei einer bevorzugten Ausführungsform weisen die Aluminiumplättchen weiterhin einen Quecksilbergehalt von bevorzugt ≤ 1 ppm, einen Arsengehalt von bevorzugt ≤ 2 ppm, einen Bleigehalt von bevorzugt ≤ 10 ppm, einen Cadmiumgehalt von bevorzugt ≤ 1 ppm, einen Bariumgehalt von bevorzugt ≤ 10 ppm, einen Chromgehalt von bevorzugt ≤ 20 ppm, einen Nickelgehalt von bevorzugt ≤ 20 ppm, einen Kupfergehalt von bevorzugt ≤ 20 ppm, einen Cobaltgehalt von bevorzugt ≤ 20 ppm, einen Antimongehalt von bevorzugt ≤ 2 ppm, einen Selengehalt von bevorzugt ≤ 10 ppm und einen Zinkgehalt von bevorzugt ≤ 20 ppm auf.

Werden Kupferplättchen als metallisches plättchenförmiges Substrat eingesetzt, weisen diese bevorzugt einen Kupfergehalt von ≥ 95 Gew.-%, weiter bevorzugt von ≥ 96 Gew.-%, besonders bevorzugt von ≥ 97 Gew.-% und ganz besonders bevorzugt von ≥ 98 Gew.-%, jeweils bezogen auf das Gesamtgewicht des Kupferplättchens, auf. Bei einer bevorzugten Ausführungsform weisen die Kupferplättchen weiterhin einen Quecksilbergehalt bevorzugt von ≤ 1 ppm, einen Arsengehalt bevorzugt von ≤ 3 ppm, einen Bleigehalt bevorzugt von ≤ 20 ppm, einen Cadmiumgehalt bevorzugt von ≤ 15 ppm, einen Bariumgehalt bevorzugt von ≤ 10 ppm, einen Chromgehalt bevorzugt von ≤ 20 ppm, einen Nickelgehalt bevorzugt von ≤ 20 ppm, einen Cobaltgehalt bevorzugt von ≤ 20 ppm, einen Antimongehalt bevorzugt von ≤ 2 ppm und einen Selengehalt bevorzugt von ≤ 10 ppm auf.

Werden Goldbronzeplättchen als metallisches plättchenförmiges Substrat eingesetzt, weisen diese bevorzugt einen Kupfergehalt aus einem Bereich von 70 Gew.-% bis 95 Gew.-%, einen Zinkgehalt aus einem Bereich von < 5 Gew.-% bis < 30 Gew.-%, einen Aluminiumgehalt aus einem Bereich von 0,01 Gew.-% bis ≤ 1,5 Gew.-%, einen Zinngehalt aus einem Bereich von 0,001 Gew.-% bis ≤ 0,5 Gew.-%, jeweils bezogen auf das Gesamtgewicht der Goldbronzeplättchen, auf. Bei einer bevorzugten Ausführungsform weisen die Goldbronzeplättchen weiterhin einen Quecksilbergehalt bevorzugt von ≤ 1 ppm, einen Arsengehalt bevorzugt von ≤ 3 ppm, einen Bleigehalt bevorzugt von ≤ 20 ppm, einen Cadmiumgehalt bevorzugt von ≤ 15 ppm, einen Bariumgehalt bevorzugt von ≤ 10 ppm, einen Chromgehalt bevorzugt von ≤ 20 ppm, einen Nickelgehalt bevorzugt von ≤ 20 ppm, einen Cobaltgehalt bevorzugt von ≤ 20 ppm, einen Antimongehalt bevorzugt von ≤ 2 ppm und einen Selengehalt bevorzugt von ≤ 10 ppm auf.

Werden Eisenplättchen als metallisches plättchenförmiges Substrat eingesetzt, werden diese vorzugsweise aus reduzierend behandeltem Carbonyleisenpulver gemäß Hauptanspruch der EP 1 251 152 A1 hergestellt.

Bei einer weiteren Ausführungsform sind die als metallisches plättchenförmiges Substrat verwendbaren Aluminium- oder Aluminiumlegierungsplättchen gemäß Hauptanspruch der WO 96/38505 A1 nasschemisch oxidiert und weisen optional eine hochbrechende Metallchalcogenidschicht gemäß Hauptanspruch der WO 2005/049739 A2 auf.

Bei einer weiteren Ausführungsform umfassen die in der erfindungsgemäßen Nagellackkomposition zu verwendenden oberflächenmodifizierten Effektpigmente kommerziell erhältliche Leafing-Aluminiumeffektpigmente, wie beispielsweise Eternabrite Premier 251, Eternabrite Premier 255 oder Eternabrite Premier 351, jeweils Fa. Silberline. Bei einer bevorzugten Ausführungsform umfassen die in der erfindungsgemäßen Nagellackkomposition zu verwendenden oberflächenmodifizierten Effektpigmente als metallisches plättchenförmiges Substrat durch PVD-Verfahren hergestellte Aluminiumplättchen oder Metalleffektpigmente gemäß dem Hauptanspruch der WO 2010/086165 A1, wie beispielsweise METALURE A41010 AE, METALURE A41506 EN, METALURE A 41510 EN, METALURE A31017 AE, METALURE A31010 BG, METALURE A31010 AE, METALURE A21010 AE, METALURE L55350AE, METALURE L63418, METALURE L 55700, METALURE A 61010 BG, METALURE A61010 AE, METALURE A61006 AE, METALURE A61006 BG, METALURE A41010 BG oder METALURE L71011AE, alle Fa. ECKART GmbH.

Die mittlere Dicke $h_{50}$ der optional zu beschichtenden metallischen plättchenförmigen Substrate liegt bevorzugt in einem Bereich von 10 nm bis 2000 nm, weiter bevorzugt in einem Bereich von 13 nm bis 1500 nm, besonders bevorzugt in einem Bereich von 15 nm bis 900 nm und ganz besonders bevorzugt in einem Bereich von 18 nm bis 600 nm. Unter der mittleren Dicke $h_{50}$ wird erfindungsgemäß das der Medianwert der Summenverteilungskurve der Dickenverteilung verstanden, sofern nicht anders angegeben.

Die Bestimmung von $h_{50}$ erfolgte bevorzugt gemäß der in der WO 2004/087816 A2 (Seiten 24 und 25) beschriebenen Methode mittels REM.

Der $h_{50}$-Wert der Summenhäufigkeitsverteilung der Dickenverteilungsfunktion, wie sie bevorzugt durch Auszählung im REM erhalten wird, gibt an, dass 50% der vermessenen oberflächenmodifizierten Effektpigmente eine Dicke aufweisen, der gleich oder kleiner als der jeweils angegebene Wert ist.

Bei weiter bevorzugten Ausführungsformen werden als metallisches plättchenförmiges Substrat nassvermahlene Aluminiumplättchen mit einem Span der Dickenverteilung $\Delta h = (h_{90}-h_{10})/h_{50}$ aus einem Bereich von 30 bis 140 % und bevorzugt aus einem Bereich von 30 bis 70 % und ganz besonders bevorzugt aus einem Bereich von 30 bis 50 % eingesetzt. Die Herstellung derartiger Metalleffektpigmente sind in der WO 2004/087816 A2 oder der WO 2008/077612 A2 beschrieben, die hiermit unter Bezugnahme aufgenommen sind.

Bei einer weiteren Ausführungsform werden als metallisches plättchenförmiges Substrat Kupferplättchen oder Goldbronzeplättchen mit einer Dickenverteilung, charakterisiert durch einen $h_{50}$-Wert von 10 bis 50 nm, und einem $h_{90}$-Wert von 20 bis 70 nm. Die Herstellung derartiger metallischer Effektpigmente kann der WO 2009/152941 A2 entnommen werden.

Bei einer weiteren Ausführungsform beträgt die relative Standardabweichung der Dickenverteilung der metallischen plättchenförmigen Substrate 11% bis 98%, bevorzugt 22% bis 78%, besonders bevorzugt 28% bis 68% und ganz besonders bevorzugt 34% bis 64%. Die relative Standardabweichung in [%] ist dabei der Quotient aus berechneter Standardabweichung der Dickenverteilung und mittlerer Dicke $h_{50}$.

Die in der erfindungsgemäßen Nagellackkomposition zu verwendenden, oberflächenmodifizierten Effektpigmente basierend auf metallischen, nicht beschichteten, plättchenförmigen Substraten können optional wenigstens eine Schicht aus oder mit wenigstens einem Metalloxid, Metallhydroxid und/oder Metalloxidhydrat aufweisen, wobei das Metallion ausgewählt aus der Gruppe der Metalle, bestehend aus Al, Si, Sn, Zn Ti und Fe, bevorzugt ausgewählt aus der Gruppe de Metalle, bestehend aus Al und Si, ist. Vorstehend aufgeführte Metalloxide, Metallhydroxide und/oder Metalloxidhydrate können als (i) separate Schichten, (ii) gemischtes Metalloxid, gemischtes Metallhydroxid und/oder gemischtes Metalloxidhydrat oder (iii) in separaten Schichten auf dem metallischen plättchenförmigen Substrat vorliegen.

Die in der erfindungsgemäßen Nagellackkomposition zu verwendenden, oberflächenmodifizierten Effektpigmente basierend auf metallischen plättchenförmigen Substraten können eine beliebige mittlere Teilchengröße $D_{50}$ aufweisen. Die Dso-Werte der erfindungsgemäßen, bevorzugt in einer Nagellackkomposition zu verwendenden, oberflächenmodifizierten Effektpigmente basierend auf metallischen plättchenförmigen Substraten liegen bevorzugt in einem Bereich von 2 $\mu$m bis 150 $\mu$m, weiter bevorzugt in einem Bereich von 2,5 $\mu$m bis 170 $\mu$m, weiter bevorzugt in einem Bereich von 3 $\mu$m bis 140 $\mu$m, besonders bevorzugt in einem Bereich von 3,5 $\mu$m bis 90 $\mu$m und ganz besonders bevorzugt in einem Bereich von 3,8 $\mu$m bis 56 $\mu$m. Äußerst bevorzugt weisen die erfindungsgemäßen, bevorzugt in einer Nagellackkomposition zu verwendenden, oberflächenmodifizierten Effektpigmente basierend auf metallischen plättchenförmigen Substraten einen Dso-Wert aus einem Bereich 2,5 $\mu$m bis 14 $\mu$m oder aus einem Bereich von 15 $\mu$m bis 35 $\mu$m auf. Unter 2 $\mu$m nehmen die Glanzwerte deutlich ab und oberhalb von 150 $\mu$m erhält man keine einheitlich geschlossene metallische Oberfläche.

Bevorzugt weisen die metallischen plättchenförmigen Substrate, die durch Nassvermahlung hergestellt werden und eine mittlere Dicke $h_{50}$ unter 100 nm aufweisen, einen Dso-Wert aus einem Bereich von 8 $\mu$m bis 25 $\mu$m auf.

Die $D_{10}$-Werte der in der erfindungsgemäßen Nagellackkomposition zu verwendenden, oberflächenmodifizierten Effektpigmente basierend auf metallischen plättchenförmigen Substraten liegen bevorzugt in einem Bereich von 1 $\mu$m bis 60 $\mu$m, weiter bevorzugt in einem Bereich von 2 $\mu$m bis 40 $\mu$m, besonders bevorzugt in einem Bereich von 4 $\mu$m bis 31 $\mu$m und ganz besonders bevorzugt in einem Bereich von 5 $\mu$m bis 19 $\mu$m. Unter 1 $\mu$m nehmen die Glanzwerte

deutlich ab und oberhalb von 60 µm erhält man keine einheitlich geschlossene metallische Oberfläche.

Die $D_{90}$-Werte der in der erfindungsgemäßen Nagellackkomposition zu verwendenden, oberflächenmodifizierten Effektpigmente basierend auf metallischen plättchenförmigen Substraten liegen bevorzugt in einem Bereich von 10 µm bis 600 µm, weiter bevorzugt in einem Bereich von 30 µm bis 200 µm, besonders bevorzugt in einem Bereich von 40 µm bis 150 µm und ganz besonders bevorzugt in einem Bereich von 45 µm bis 120 µm. Unter 10 µm nehmen die Glanzwerte deutlich ab und oberhalb von 600 µm erhält man keine einheitlich geschlossene metallische Oberfläche.

Bei einer Ausführungsform besitzen die in der erfindungsgemäßen Nagellackkomposition zu verwendenden, oberflächenmodifizierten Effektpigmente basierend auf metallischen plättchenförmigen Substraten einen Span $\Delta D$, definiert als $\Delta D = \frac{D_{90} - D_{10}}{D_{50}}$, aus einem Bereich von 0,7 bis 2,5, bevorzugt aus einem Bereich von 0,8 bis 2,2, weiter bevorzugt aus einem Bereich von 0,9 bis 1,9, besonders bevorzugt aus einem Bereich von 0,9 bis 1,8 und ganz besonders bevorzugt aus einem Bereich von 1 bis 1,7.

Bei einer weiteren Ausführungsform weisen die in der erfindungsgemäßen Nagellackkomposition zu verwendenden, oberflächenmodifizierten Effektpigmente basierend auf metallischen plättchenförmigen Substraten ein Aspektverhältnis, definiert als Quotient aus $D_{50}$ und mittlerer Dicke, bevorzugt aus einem Bereich von 10 bis 2000, weiter bevorzugt aus einem Bereich von 30 bis 1200, besonders bevorzugt aus einem Bereich von 100 bis 1100 und ganz besonders bevorzugt aus einem Bereich von 200 bis 1000 auf. Unterhalb der Untergrenzen sind die optischen Eigenschaften der metallischen Effektpigmente noch nicht ausreichend zur Erreichung eines Spiegelglanzes und oberhalb von 2000 sind die metallischen Effektpigmente nur sehr schwer herstellbar.

Bei einer weiteren Ausführungsform weisen die in der erfindungsgemäßen Nagellackkomposition zu verwendenden, oberflächenmodifizierten Effektpigmente basierend auf metallischen plättchenförmigen Substraten bevorzugt eine mittlere Gesamtdicke $h_{50}$ aus einem Bereich von 20 nm bis 4000 nm, weiter bevorzugt aus einem Bereich von 30 nm bis 3000 nm, besonders bevorzugt aus einem Bereich von 70 nm bis 2000 nm und ganz besonders bevorzugt aus einem Bereich von 230 nm bis 1300 nm auf. Unter mittlerer Gesamtdicke wird die komplette mittlere Dicke des oberflächenmodifzierten Effektpigments, also metallisches plättchenförmiges Substrat plus optionale Beschichtung plus Oberflächenmodifizierung, verstanden.

Bei einer bevorzugten Ausführungsform umfassen die in der erfindungsgemäßen Nagellackkomposition zu verwendenden, oberflächenmodifizierten Effektpigmente als metallisches plättchenförmiges Substrat Metallplättchen hergestellt durch ein PVD-Verfahren mit einem Dso-Wert aus einem Bereich von 2,5 µm bis 90 µm, weiter bevorzugt aus einem Bereich von 8 µm bis 25 µm und einer mittleren Dicke aus einem Bereich von 13 nm bis 60 nm eingesetzt. Bei dieser Ausführungsform werden die Aluminiumplättchen vorzugsweise nicht beschichtet. Die auf Aluminiumplättchen vorhandene natürliche Oxidschicht wird hierbei nicht als aufgebrachte Beschichtung verstanden. Überraschenderweise können auch Spiegelglanzeffekte mit sehr kleinen PVD-Pigmenten erreicht werden. Diese Pigmente können z.B. hergestellt werden, indem man gewöhnliche PVD-Pigmente weiter vermahlt oder durch Ultraschalleinwirkung zerkleinert.

Besonders bevorzugt handelt es sich bei diesen Metallplättchen um Aluminiumplättchen. Besonders bevorzugt weisen diese Aluminium-PVD Pigmente einen $D_{50}$-Wert aus einem Bereich von 6 µm bis 18 µm und einer mittleren Dicke aus einem Bereich von 14 nm bis 40 nm auf. Ganz besonders bevorzugt sind Aluminium-PVD Pigmente mit einen Dso-Wert aus einem Bereich von 7 µm bis 16 µm und einer mittleren Dicke aus einem Bereich von 15 nm bis 35 nm.

In weiteren Ausführungsformen werden nassvermahlene Metallplättchen und insbesondere Aluminiumplättchen verwendet, die eine ähnliche mittlere Dicke wie PVD-Pigmente aufweisen.

So sind bevorzugt nassvermahlene Metallplättchen und insbesondere Aluminiumplättchen, mit einer mittleren Dicke $h_{50}$ aus einem Bereich von 20 bis unter 100 nm und ganz besonders bevorzugt einer mittleren Dicke $h_{50}$ aus einem Bereich von 25 bis 60 nm.

Mit derartigen Metalleffektpigmenten lassen sich auch Spiegelglanzeffekte erzielen, wenn gleich in der Regel durch PVD-Verfahren hergestellte Metallpigmente noch bessere Ergebnisse liefern.

Die nichtmetallischen plättchenförmigen Substrate können aus der Gruppe, bestehend aus natürlichen Glimmerplättchen, synthetischen Glimmerplättchen, Glasplättchen, $SiO_2$-Plättchen, $Al_2O_3$-Plättchen, Kaolinplättchen, Talkplättchen und Bismuthoxychloridplättchen, ausgewählt werden. Die in der erfindungsgemäßen Nagellackkomposition zu verwendenden, oberflächenmodifizierten Effektpigmente bzw. die oberflächenmodifizierten Effektpigmente gemäß Anspruch 3 können auch auf Gemischen der vorstehend angegebenen nichtmetallischen plättchenförmigen Substrate basieren. Die vorgenannten nichtmetallischen plättchenförmigen Substrate können weiterhin eine oder mehrere Schichten aus oder mit wenigstens einem hoch- und/oder niedrigbrechendem Metalloxid, Metallhydroxid und/oder Metalloxidhydrat aufweisen und kalziniert sein. So können als Substrate also auch Perlglanzpigmente oder Interferenzpigmente verwendet werden.

Bevorzugt werden die nichtmetallischen plättchenförmigen Substrate aus der Gruppe, bestehend aus natürlichen Glimmerplättchen, synthetischen Glimmerplättchen, Glasplättchen, $SiO_2$-Plättchen, $Al_2O_3$-Plättchen und deren Gemische, ausgewählt. Besonders bevorzugt werden die nichtmetallischen plättchenförmigen Substrate aus der Gruppe, bestehend aus natürlichen Glimmerplättchen, synthetischen Glimmerplättchen, Glasplättchen und deren Gemische, ausgewählt. Ganz besonders bevorzugt sind als nichtmetallische plättchenförmige Substrate synthetische Glimmerplättchen und/oder Glasplättchen. Insbesondere Glasplättchen sind als nichtmetallisches plättchenförmiges Substrat bevorzugt.

Die als nichtmetallisches plättchenförmiges Substrat verwendbaren Glasplättchen können im Hinblick auf ihre Zusammensetzung aus Silikatglas, wie Kalknatronglas, Bleikristallglas, E-Glas, A-Glas, C-Glas, ECR-Glas, Duranglas, Fensterglas, Laborglas, Aluminosilikatglas oder Borosilikatglas bestehen. Bevorzugt weisen die Glasplättchen eine Zusammensetzung entsprechend der Lehre, insbesondere entsprechend dem Hauptanspruch, der EP 1 980 594 B1, besonders bevorzugt entsprechend der Lehre, insbesondere entsprechend dem jeweiligen Hauptanspruch, der EP 1 829 833 B1 oder der EP 2 042 474 B1, auf. Die Herstellung der als nichtmetallisches plättchenförmiges Substrat einsetzbaren Glasplättchen erfolgt vorzugsweise nach dem in EP 289 240 B1 beschriebenen Verfahren.

Bei einer Ausführungsform können die Glasplättchen durch den Zusatz von wenigstens einem anorganischen Farbmittel bei ihrer Herstellung gezielt eingefärbt werden. Geeignete Farbmittel sind solche, die sich bei der jeweiligen Schmelztemperatur der Glaszusammensetzung nicht zersetzen. Der Anteil an Farbmittel liegt hierbei bevorzugt in einem Bereich von insgesamt 0,1 Gew.-% bis 20 Gew.-%, besonders bevorzugt in einem Bereich von insgesamt 0,2 Gew.-% bis 15 Gew.-% und ganz besonders bevorzugt in einem Bereich von insgesamt 0,5 Gew.-% bis 10 Gew.-%, jeweils bezogen auf das Gesamtgewicht der Glaszusammensetzung. Geeignete Farbmittel sind insbesondere elementare Edelmetalle, wie Au, Pd oder Pt, die Kationen oder komplexe Anionen der Elemente Cu, Cr, Mn, Fe, Ti und/oder Co sowie Mischungen der vorstehend aufgeführten Farbmittel.

Bei einer weiteren Ausführungsform liegt der Brechungsindex der als nichtmetallisches plättchenförmiges Substrat einsetzbaren Glasplättchen in einem Bereich von 1,45 bis 1,80, vorzugsweise in einem Bereich von 1,50 bis 1,70.

Bei einer weiteren Ausführungsform können die nichtmetallischen plättchenförmigen Substrate, insbesondere Glasplättchen, mit einer Schicht, die Siliziumoxid, Siliziumhydroxid, Siliziumoxidhydrat umfasst oder daraus besteht, umhüllt sein. Beispielsweise kann durch die vorgenannte Beschichtung bei Verwendung von Glasplättchen die Glasoberfläche vor chemischer Veränderung, wie Quellung, Auslaugen von Glasbestandteilen oder Auflösung in aggressiven sauren Belegungslösungen, geschützt werden.

Die als nichtmetallisches plättchenförmiges Substrat verwendbaren synthetischen Glimmerplättchen können eine Zusammensetzung gemäß Hauptanspruch der CN 102718229 A oder gemäß Hauptanspruch der US 2014/0251184 A1 aufweisen. Sie können weiterhin gemäß den Angaben in der EP 0 723 997 A1, Seite 3 bis Seite 4, hergestellt werden.

Bei den als nichtmetallisches plättchenförmiges Substrat verwendbaren synthetischen Glimmerplättchen handelt es sich vorzugsweise um plättchenförmigen Fluorphlogopit der Formel $KMg_3AlSi_3O_{10}F_2$, $KMg_{2½}(Si_4O_{10})F_2$ oder $NaMg_{2½}(Si_4O_{10})F_2$, insbesondere um plättchenförmigen Fluorphlogopit der Formel $KMg_3AlSi_3O_{10}F_2$, welcher gemäß Röntgenfluoreszenzanalyse (RFA) vorzugsweise die in Tabelle 1 genannten Bestandteile als jeweiliges Metalloxid in den dort aufgeführten Bereichen umfasst.

Tabelle 1: Bevorzugte Zusammensetzungen von synthetischen Glimmerplättchen gemäß RFA

| Zusammensetzung synthetischer Glimmerplättchen, Angaben in Gew.-%, jeweils bezogen auf das Gesamtgewicht der synthetischen Glimmerplättchen | |
|---|---|
| $SiO_2$ | 38 bis 46 |
| $Al_2O_3$ | 10 bis 14 |
| $K_2O$ | 9 bis 13 |
| $Fe_2O_3$ | 0,01 bis 0,25 |
| MgO | 26 bis 34 |
| MnO | 0 bis 0,05 |
| $Na_2O$ | 0 bis 13 |

Die mittlere Dicke $h_{50}$ der optional zu beschichtenden nichtmetallischen plättchenförmigen Substrate liegt bevorzugt in einem Bereich von 50 nm bis 5000 nm, besonders bevorzugt in einem Bereich von 60 nm bis 3000 nm und ganz besonders bevorzugt in einem Bereich von 70 nm bis 2000 nm.

Bei einer Ausführungsform liegt die mittlere Dicke $h_{50,Glas}$ für Glasplättchen als optional zu beschichtendes nichtmetallisches plättchenförmiges Substrat in einem Bereich von 750 nm bis 1500 nm, bevorzugt in einem Bereich von 850 nm bis 1400 nm und besonders bevorzugt in einem Bereich von 900 nm bis 1300 nm. Dünnere nichtmetallische plättchenförmige Substrate führen zu einer geringeren Gesamtdicke der optional zu beschichtenden, in der erfindungsgemäßen Nagellackkomposition zu verwendenden, oberflächenmodifzierten Effektpigmente. So sind als nichtmetallisches plättchenförmiges Substrat ebenfalls bevorzugt Glasplättchen, deren mittlere Dicke $h_{50,Glas}$ in einem Bereich von 50 nm bis 700 nm, weiter bevorzugt in einem Bereich von 101 nm bis 600 nm, besonders bevorzugt in einem Bereich von 160 nm bis 500 nm und ganz besonders bevorzugt in einem Bereich 200 nm bis 400 nm liegt.

Bei einer weiteren Ausführungsform liegt die mittlere Dicke $h_{50,Glimmer}$ der natürlichen oder synthetischen Glimmerplättchen als optional zu beschichtendes nichtmetallisches plättchenförmiges Substrat bevorzugt in einem Bereich von 80 nm bis 1300 nm, weiter bevorzugt in einem Bereich von 90 nm bis 1000 nm, besonders bevorzugt in einem Bereich von 99 nm bis 800 nm und ganz besonders bevorzugt in einem Bereich von 200 nm bis 600 nm.

Beschichtet man nichtmetallische plättchenförmige Substrate unterhalb einer mittleren Dicke $h_{50}$ von 50 nm mit beispielsweise hochbrechenden Metalloxiden, so werden extrem bruchempfindliche Effektpigmente erhalten, die schon beim Einarbeiten in das jeweilige Anwendungsmedium zerbrechen können, was wiederum eine signifikante Herabsetzung des Glanzes bedingt.

Oberhalb einer mittleren Dicke des nichtmetallischen plättchenförmigen Substrats von 5000 nm können die Effektpigmente insgesamt zu dick werden. Damit geht eine schlechtere spezifische Deckfähigkeit einher, d.h. die abgedeckte Fläche pro Gewichtseinheit an Effektpigment ist geringer. Außerdem orientieren sich derartig dicke Effektpigmente in geringerem Ausmaß planparallel zum Untergrund im Anwendungsmedium. Aus einer schlechteren Orientierung wiederum resultiert ein verminderter Glanz. Auch im Hinblick auf die Haptik können insgesamt zu dicke Effektpigmente unvorteilhaft in einer Anwendung sein.

Bei einer Ausführungsform beträgt die relative Standardabweichung der Dickenverteilung der nichtmetallischen plättchenförmigen Substrate 15% bis 100%, bevorzugt 17% bis 70%, besonders bevorzugt 19% bis 61% und ganz besonders bevorzugt 21% bis 41%. Die relative Standardabweichung in [%] ist dabei der Quotient aus berechneter Standardabweichung und mittlerer Dicke $h_{50}$.

Die nichtmetallischen plättchenförmigen Substrate können optional mit wenigstens einem Metalloxid, Metallhydroxid und/oder Metalloxidhydrat beschichtet werden. Diese optionale Beschichtung kann wenigstens eine hochbrechende und/oder wenigstens eine niedrigbrechende Schicht umfassen. Unter einer hochbrechenden Schicht wird eine Schicht mit Brechungsindex von $n \geq 1{,}8$, bevorzugt von $n \geq 2{,}0$ und besonders bevorzugt von $n \geq 2{,}2$ verstanden. Unter einer niedrigbrechenden Schicht wird eine Schicht mit Brechungsindex von $n < 1{,}8$ und bevorzugt von $n < 1{,}6$ verstanden. Für die Zuordnung in hochbrechende Schicht und in niedrigbrechende Schicht werden literaturbekannte Brechungsindexe herangezogen.

Die optional vorhandene Beschichtung kann wenigstens eine hochbrechende Schicht aus oder mit wenigstens einem Metalloxid, Metallhydroxid und/oder Metalloxidhydrat umfassen, wobei das Metallion aus der Gruppe der Metalle, bestehend aus Ti, Fe, Sn, Mn, Zr, Ca, Sr, Ba, Ni, Ag, Zn, Cu, Ce, Cr und Co, ausgewählt ist, bevorzugt ausgewählt aus der Gruppe der Metalle, bestehend aus Ti, Fe, Sn, Zr, Ag, Zn, Cu, Ce und Cr, und besonders bevorzugt ausgewählt aus der Gruppe der Metalle, bestehend aus Ti, Fe und Sn, ist. Vorstehend aufgeführte Metalloxide, Metallhydroxide und/oder Metalloxidhydrate können als (i) Mischschicht, (ii) gemischtes Metalloxid, gemischtes Metallhydroxid und/oder gemischtes Metalloxidhydrat, vorzugsweise als Eisentitanat, wie Ilmenit, Pseudobrookit oder Pseudorutil, oder (iii) in separaten Schichten auf dem nichtmetallischen plättchenförmigen Substrat vorliegen.

Alternativ oder zusätzlich zu der vorstehend erwähnten wenigstens einen hochbrechenden Schicht kann die optional vorhandene Beschichtung wenigstens eine niedrigbrechende Schicht aus oder mit wenigstens einem Metalloxid, Metallhydroxid und/oder Metalloxidhydrat umfassen, wobei das Metallion aus der Gruppe der Metalle, bestehend aus Si, Al und B ausgewählt ist. Sofern hochbrechende und niedrigbrechende Schichten in der optional vorhandenen Beschichtung vorhanden sind, ist es bevorzugt, dass diese alternierend vorliegen.

Bei einer Ausführungsform kann die wenigstens eine hochbrechende und/oder die wenigstens eine niedrigbrechende Schicht dotiert sein, wobei die Dotierung Metalloxide, Metallhydroxide und/oder Metalloxidhydrate umfassen kann, deren Metallion aus der Gruppe der Metalle, bestehend aus Al, Ce, Zr und Sn, bevorzugt bestehend aus Al, Zr und Sn, ausgewählt ist. Der Anteil an Dotierung liegt bevorzugt bei insgesamt $\leq 1$ Gew.-%, besonders bevorzugt bei insgesamt $\leq 0{,}5$ Gew.-% und ganz besonders bevorzugt bei insgesamt $\leq 0{,}2$ Gew.-%, jeweils bezogen auf das Gesamtgewicht der oberflächenmodifizierten Effektpigmente.

Bei einer weiteren Ausführungsform kann die optional vorhandene Beschichtung alternativ oder zusätzlich zu der wenigstens ein Metalloxid, Metallhydroxid und/oder Metallhydroxid umfassenden Schicht wenigstens eine semitransparente Metallschicht umfassen. Die Metalle der semitransparenten Metallschicht können aus der Gruppe, bestehend aus Ag, Al, Cr, Ni, Au, Pt, Pd, Cu, Zn und Ti, bevorzugt ausgewählt aus der Gruppe, bestehend aus Ag, Au und Cu, ausgewählt sein. Selbstverständlich kann die semitransparente Metallschicht auch Legierungen oder Mischungen der vorstehend aufgeführten Metalle umfassen. Die mittlere Dicke der semitransparenten Metallschicht liegt bevorzugt in einem Bereich von 1 nm bis 30 nm, besonders bevorzugt in einem Bereich von 4 nm bis 26 nm und besonders bevorzugt in einem Bereich von 7 nm bis 21 nm.

Bei einer bevorzugten Ausführungsform weisen die nichtmetallischen plättchenförmigen Substrate wenigstens eine der vorstehend beschriebenen Schichten, besonders bevorzugt wenigstens eine Schicht aus oder mit hochbrechenden Metalloxiden, Metallhydroxiden und/oder Metalloxidhydraten auf.

Bei einer weiteren Ausführungsform besitzen die in der erfindungsgemäßen Nagellackkomposition zu verwendenden, oberflächenmodifizierten Effektpigmente bzw. die oberflächenmodifizierten Effektpigmente gemäß Anspruch 3, jeweils basierend auf nichtmetallischen plättchenförmigen Substraten einen Span $\Delta D$, definiert als $\Delta D = \frac{D_{90} - D_{10}}{D_{50}}$, aus einem Bereich von 0,7 bis 2,0, bevorzugt aus einem Bereich von 0,7 bis 1,5, weiter bevorzugt aus einem Bereich von 0,8 bis 1,3, besonders bevorzugt aus einem Bereich von 0,8 bis 1,2 und ganz besonders bevorzugt aus einem Bereich von 0,85 bis 1,1. Die Vorteile einer engen Größenklassierung in Bezug auf Farbreinheit und/oder Glanz der resultierenden Effektpigmente werden beispielsweise in EP 2 217 664 A1, EP 2 346 950 A1, EP 2 356 181 A1, EP 2 346 949 A1, EP 2 367 889 A1 beschrieben.

Die in der erfindungsgemäßen Nagellackkomposition zu verwendenden, oberflächenmodifizierten Effektpigmente basierend auf nichtmetallischen plättchenförmigen Substraten weisen eine mittlere Teilchengröße $D_{50}$ aus einem Bereich von 2 µm bis 360 µm auf. Die Dso-Werte der in der erfindungsgemäßen Nagellackkomposition zu verwendenden, oberflächenmodifizierten Effektpigmente basierend auf nichtmetallischen plättchenfömigen Substraten liegen bevorzugt in einem Bereich von 3 µm bis 350 µm, weiter bevorzugt in einem Bereich von 4 µm bis 211 µm, weiter bevorzugt in einem Bereich von 6 µm bis 147 µm, besonders bevorzugt in einem Bereich von 7 µm bis 99 µm und ganz besonders bevorzugt in einem Bereich von 8 µm bis 56 µm. Äußerst bevorzugt weisen die in der erfindungsgemäßen Nagellackkomposition zu verwendenden, oberflächenmodifizierten Effektpigmente basierend auf nichtmetallischen plättchenförmigen Substraten einen $D_{50}$-Wert aus einem Bereich von 3 bis 15 µm oder aus einem Bereich von 10 bis 35 µm oder aus einem Bereich von 25 bis 45 µm oder aus einem Bereich von 30 bis 65 µm oder aus einem Bereich von 40 bis 140 µm oder aus einem Bereich von 135 bis 250 µm auf.

Die $D_{10}$-Werte der in der erfindungsgemäßen Nagellackkomposition zu verwendenden, oberflächenmodifizierten Effektpigmente basierend auf nichtmetallischen plättchenförmigen Substraten liegen bevorzugt in einem Bereich von 1 bis 120 µm.

Besonders bevorzugt liegen die $D_{10}$-Werte der erfindungsgemäßen, bevorzugt in einer Nagellackkomposition zu verwendenden, oberflächenmodifizierten Effektpigmente basierend auf nichtmetallischen plättchenförmigen Substraten in einem Bereich von 1 µm bis 5 µm oder in einem Bereich von 5 µm bis 25 µm oder in einem Bereich von 10 µm bis 30 µm oder in einem Bereich von 20 µm bis 45 µm oder in einem Bereich von 25 µm bis 65 µm oder in einem Bereich von 75 bis 110 µm.

Die $D_{90}$-Werte der in der erfindungsgemäßen Nagellackkomposition zu verwendenden, oberflächenmodifizierten Effektpigmente basierend auf nichtmetallischen plättchenförmigen Substraten liegen bevorzugt in einem Bereich von 6 bis 500 µm. Besonders bevorzugt liegen die $D_{90}$-Werte der erfindungsgemäßen, bevorzugt in einer Nagellackkomposition zu verwendenden, oberflächenmodifizierten Effektpigmente basierend auf nichtmetallischen plättchenförmigen Substraten in einem Bereich von 8 µm bis 250 µm oder in einem Bereich von 10 µm bis 150 µm oder in einem Bereich von 40 µm bis 70 µm oder in einem Bereich von 68 µm bis 110 µm oder in einem Bereich von 120 µm bis 180 µm oder in einem Bereich von 400 µm bis 490 µm.

Bei einer besonders bevorzugten Ausführungsform umfassen die in der erfindungsgemäßen Nagellackkomposition zu verwendenden, oberflächenmodifizierte Effektpigmente als nichtmetallisches plättchenförmiges Substrat Glasplättchen mit einem $D_{50}$ Wert aus einem Bereich von 9 µm bis 390 µm, einer mittleren Dicke aus einem Bereich von 90 nm bis 590 nm, bevorzugt aus einem Bereich von 110 nm bis 340 nm, einer Glaskomposition gemäß Hauptanspruch der WO 2007/148758 A1 oder der WO 2010/024283 A1 und einer semitransparenten Schicht aus oder mit metallischem Silber.

Die mittlere Dicke des mit einer Metalloxid, Metallhydroxid und/oder einem Metalloxidhydrat beschichteten metallischen oder nichtmetallischen plättchenförmigen Substrates sowie die mittlere Gesamtdicke des oberflächenmodifizierten Effektpigments wird anhand eines gehärteten Lackfilmes, in dem die in der erfindungsgemäßen Nagellackkomposition zu verwendenden, oberflächenmodifizierten Effektpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die Dicke des metallischen oder nichtmetallischen plättchenförmigen Substrates bzw. die Gesamtdicke des oberflächenmodifizierten Effektpigments von wenigstens 100 oberflächenmodifizierten Effektpigmenten bestimmt und statistisch gemittelt wird. Mit dem Begriff "mittlere" ist erfindungsgemäß stets der $h_{50}$-Wert gemeint, sofern nicht anders angegeben.

Im Fall von in der erfindungsgemäßen Nagellackkomposition zu verwendenden, oberflächenmodifizierten Effektpigmenten, die nicht mit einem Metalloxid, Metallhydroxid und/oder einem Metalloxidhydrat beschichtet sind, erfolgt die Bestimmung der mittleren Dicke $h_{50}$ bevorzugt gemäß der in der WO 2004/087816 A2 (Seiten 24 und 25) beschriebenen Methode mittels REM.

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der vermessenen oberflächenmodifizierten Effektpigmente einen volumengemittelten Durchmesser aufweisen, der gleich oder kleiner als der jeweils angegebene Wert ist. Hierbei wird die Größenverteilungskurve der in der erfindungsgemäßen Nagellackkomposition zu verwendenden, oberflächenmodifizierten Effektpigmente sowie der oberflächenmodifizierten Effektpigmente gemäß Anspruch 3, jeweils basierend auf nichtmetallischen plättchenförmigen Substraten, mit dem Gerät Mastersizer 2000 der Fa. Malvern gemäß Herstellerangaben bestimmt. Die Auswertung der Streulichtsignale erfolgt nach der Fraunhofer-Theorie, welche auch Brechungs- und Absorptionsverhalten der Partikel beinhaltet. Die Größenverteilungskurve der in der erfindungsgemäßen Nagellackkomposition zu verwendenden, oberflächenmodifizierten Effektpigmente sowie der oberflächenmodifizierten Effektpigmente gemäß Anspruch 3, jeweils basierend auf metallischen plättchenförmigen Substraten, wird mit dem Gerät Cilas 1064 der Fa. Quantachrome oder dem Gerät Horiba LA-930 der Fa. Horiba ermittelt. Die Auswertung der Streulichtsignale erfolgt nach der Fraunhofer-Theorie, welche auch Brechungs- und Absorptionsverhalten der Partikel beinhaltet.

Es hat sich gezeigt, dass die erfindungsgemäß oberflächenmodifizierten Effektpigmente nicht in allen Nagellacksystemen, wie sie zum Beispiel in der EP 1796794 B2 offenbart wurden, gute Ergebnisse zeigt. Häufig geht der Leafing-Effekt bei der Applikation oder danach verloren und damit einhergehend kommen die optischen Eigenschaften der Effektpigmente nicht vollkommen zur Geltung.

Daher ist ein wesentlicher Bestandteil der vorliegenden Erfindung die Bereitstellung eines Nagellacks, in dem die leafing-Eigenschaften der oberflächenmodifizierten Effektpigmente sehr gut zur Geltung kommen.

Die Erfindung richtet sich daher auf eine Nagellackkomposition enthaltend

a) wenigstens ein mit einem Ausgangsmaterial (Additiv) oberflächenmodifiziertes Effektpigment wobei das Effektpigment ein plättchenförmiges Substrat und optional wenigstens eine auf dem Substrat aufgebrachte Beschichtung umfasst

b) wenigstens ein kohlenwasserstoffhaltiges Harz und

c) wenigstens ein Lösemittel oder Lösemittelgemisch,

wobei als Ausgangsmaterial (Additiv) zur Oberflächenmodifizierung des Effektpigments wenigstens eine Verbindung aus der Gruppe bestehend aus phosphorsäureesterhaltigen, phosphonsäureesterhaltigen, phosphonsäurehaltigen, fettsäurehaltigen und/oder silanhaltigen Verbindungen oder Mischungen hiervon, entnommen wird.

Bei den zur Oberflächenmodifizierung einsetzbaren Phosphorsäureestern und/oder Phosphorsäuren und/oder Phosphonsäureestern und/oder Phosphonsäuren kann es sich um Phosphorsäureester der allgemeinen Formel (R-O)$_n$-P(O)(OR')(OR")$_m$ bzw. Phosphonsäureester der allgemeinen Formel R-P(O)(OR)(OR") handeln, wobei die Reste R, R' und R" bevorzugt folgende Bedeutung haben:

R = linearer und/oder verzweigter Alkylrest mit einer Kohlenstoffkette aus einem Bereich von $C_{10}$ bis $C_{20}$ und R' = R" = H, linearer und/oder verzweigter Alkylrest mit einer Kohlenstoffkette aus einem Bereich von $C_1$ bis $C_6$, bevorzugt $C_1$ bis $C_3$, wobei R' und R" identisch oder verschieden voneinander sein können und wobei n = 1 oder 2 und m = n - 1 und n + m = 2 ist.

Hierbei können hinsichtlich der Zahl n sowohl reine Formen der Monoester (n = 1) oder Diester (n = 2) als auch Mischungen hiervon vorliegen.

Bei einer besonders bevorzugten Ausführungsform werden zur Oberflächenmodifzierung der in einer erfindungsgemäßen Nagellackkomposition einzusetzenden Effektpigmente primäre Phosphorsäuren oder Phosphonsäuren mit R = linearer, unsubstituierter Alkylrest mit einer Kohlenstoffkette aus einem Bereich von $C_{12}$ bis $C_{18}$, vorzugsweise aus einem Bereich von $C_{14}$ bis $C_{18}$ und R' = R" = H eingesetzt. Weiter bevorzugt ist hierbei n = 1 (Monoester).

Eine bevorzugte Phosphonsäure ist Laurylphosphonsäure und bevorzugte Phosphorsäuren sind Cetylphosphat oder Stearylphosphat.

Zur Oberflächenmodifizierung können beispielsweise 2-Ethylhexylphosphorsäureester (CAS: 12645-31-7), Laurylphosphorsäureester (CAS: 12751-23-4), Cetylphosphorsäureester (CAS: 3539-43-3), Stearylphosphorsäureester (CAS: 39471-52-8) und/oder Monoethylmono-(9Z)-9-octadecenylphosphorsäureester (CAS: 10483-96-2) eingesetzt werden.

Besonders bevorzugt sind Cetylphosphorsäureester, Stearylphosphorsäureester oder Laurylphosphorsäureester und besonders bevorzugt sind Cetylphosphorsäureester oder Stearylphosphorsäureester und ganz besonders bevorzugt sind Cetylphosphorsäureester.

Bei den zur Oberflächenmodifizierung einsetzbaren Fettsäuren kann es sich um Fettsäuren der allgemeinen Formel R-COOH handeln, wobei der Rest R bevorzugt folgende Bedeutung hat:

i. R = linearer und/oder verzweigter Alkylrest mit einer Kohlenstoffkette aus einem Bereich von $C_{12}$ bis $C_{26}$, bevorzugt aus einem Bereich von $C_{14}$ bis $C_{24}$, weiter bevorzugt aus einem Bereich von $C_{16}$ bis $C_{22}$ und besonders bevorzugt aus einem Bereich von $C_{18}$ bis $C_{20}$; oder

ii. R = linearer und/oder verzweigter Alkenylrest mit einer Kohlenstoffkette aus einem Bereich von $C_{12}$ bis $C_{26}$, bevorzugt aus einem Bereich von $C_{14}$ bis $C_{24}$, weiter bevorzugt aus einem Bereich von $C_{16}$ bis $C_{22}$ und besonders

bevorzugt aus einem Bereich von $C_{18}$ bis $C_{20}$; oder

iii. R = linearer und/oder verzweigter Alkinylrest mit einer Kohlenstoffkette aus einem Bereich von $C_{12}$ bis $C_{26}$, bevorzugt aus einem Bereich von $C_{14}$ bis $C_{24}$, weiter bevorzugt aus einem Bereich von $C_{16}$ bis $C_{22}$ und besonders bevorzugt aus einem Bereich von $C_{18}$ bis $C_{20}$.

Bei einer bevorzugten Ausführungsform werden zur Oberflächenmodifizierung der in der erfindungsgemäßen Nagellackkomposition einsetzbaren Effektpigmente Fettsäuren mit R = linearer, unsubstituierter Alkylrest mit einer Kohlenstoffkette aus einem Bereich von $C_{12}$ bis $C_{20}$, vorzugsweise aus einem Bereich von $C_{14}$ bis $C_{18}$ eingesetzt.

Bei den zur Oberflächenmodifizierung einsetzbaren Silanen kann es sich um Silane der allgemeinen Formel R-Si(OR')$_3$ handeln, wobei die Reste R und R' bevorzugt folgende Bedeutung haben:
R = linearer und/oder verzweigter Alkylrest mit einer Kohlenstoffkette aus einem Bereich von $C_6$ bis $C_{23}$, bevorzugt aus einem Bereich von $C_7$ bis $C_{20}$, besonders bevorzugt aus einem Bereich von $C_8$ bis $C_{18}$ und R' = linearer und/oder verzweigter Alkylrest mit einer Kohlenstoffkette aus einem Bereich von $C_1$ bis $C_4$, bevorzugt aus einem Bereich von $C_1$ bis $C_3$ und besonders bevorzugt aus einem Bereich von $C_1$ bis $C_2$, wobei der Alkylrest R in weiteren Ausführungsformen wenigstens einen Substituenten, ausgewählt aus der Gruppe, bestehend aus -OH, -OCH$_3$, -OC$_2$H$_5$, -NH$_2$, linearer und/oder verzweigter Alkylrest mit einer Kohlenstoffkette aus einem Bereich von $C_1$ bis $C_6$ umfassen kann.

Zur Herstellung der in der erfindungsgemäßen Nagellackkomposition zu verwendenden, oberflächenmodifizierten Effektpigmente wird das Ausgangsmaterial (Additiv) zur Oberflächenmodifizierung bevorzugt in einer Menge aus einem Bereich von 1,5 Gew.-% bis 50 Gew.-%, besonders bevorzugt aus einem Bereich von 3,2 Gew.-% bis 40 Gew.-% und ganz besonders bevorzugt aus einem Bereich von 4,8 Gew.-% bis 23 Gew.-%, jeweils bezogen auf das Gesamtgewicht des eingesetzten Effektpigments, eingesetzt. Da sich die hier angegebenen Mengen des Additivs auf das Ausgangsmaterial beziehen, kann die tatsächliche Additivmenge des fertig beschichteten Effektpigments geringer sein, da z.B. bei einer Menge von 50 Gew.-% nicht alles Additiv auf die Pigmentoberfläche aufziehen kann. Dementsprechend können auch geringere Mengen der leafing-Additive in den die Pigmente enthaltenden erfindungsgemäßen Nagellacken gefunden werden. Die recht hohen Additivmengen im Ausgangsmaterial bewirken eine sehr hohe und dichte Beschichtung der Effektpigmentoberfläche mit dem Additiv.

Bei einer besonders bevorzugten Ausführungsform wird als Ausgangsmaterial (Additiv) zur Oberflächenmodifizierung von Effektpigmenten basierend auf metallischen plättchenförmigen Substraten, die durch Nassvermahlung hergestellt wurden und eine mittlere Substratdicke $h_{50}$ aus einem Bereich von 20 nm bis 100 nm aufweisen, wenigstens eine Phosphonsäure der Formel R-P(O)(OH)$_2$ mit R = linearer Alkylrest mit einer Kohlenstoffkette aus einem Bereich von $C_8$ bis $C_{14}$, in einer Gesamtmenge bevorzugt aus einem Bereich von 4 Gew.-% bis 45 Gew.-%, weiter bevorzugt aus einem Bereich von 5 Gew.-% bis 43 Gew.-%, besonders bevorzugt aus einem Bereich von 6 Gew.-% bis 42 Gew.-% und ganz besonders bevorzugt aus einem Bereich von 9 Gew.-% bis 41 Gew.-%, jeweils bezogen auf das Gesamtgewicht des eingesetzten Effektpigments, eingesetzt.

Bei einer weiteren besonders bevorzugten erfindungsgemäßen Ausführungsform wird als Ausgangsmaterial (Additiv) zur Oberflächenmodifizierung von Effektpigmenten basierend auf metallischen plättchenförmigen Substraten, die durch PVD-Verfahren hergestellt wurden und eine mittlere Substratdicke $h_{50}$ aus einem Bereich von 13 nm bis 60 nm aufweisen, wenigstens eine Phosphonsäure der Formel R-P(O)(OH)$_2$ mit R = linearer Alkylrest mit einer Kohlenstoffkette aus einem Bereich von $C_8$ bis $C_{14}$, in einer Gesamtmenge aus einem Bereich von 5 Gew.-% bis 50 Gew.-%, weiter bevorzugt aus einem Bereich von 6 Gew.-% bis 48 Gew.-%, besonders bevorzugt aus einem Bereich von 7 Gew.-% bis 45 Gew.-% und ganz besonders bevorzugt aus einem Bereich 10 Gew.-% bis 42 Gew.-%, jeweils bezogen auf das Gesamtgewicht des eingesetzten Effektpigments, eingesetzt.

Unterhalb der angegebenen Mengen an Phosphonsäureester findet kein ausreichendes leafing der Effektpigmente statt. Oberhalb der angegebenen Mengen an Phosphonsäureester können gegebenenfalls zu große Mengen der Phosphonsäuren in die fertige Nagellackkomposition eingetragen werden.
Hierbei ist es besonders bevorzugt, dass die Effektpigmente in einem separaten Schritt mit dem Phosphonsäureester beschichtet werden, bevor sie in das Nagellacksystem eingetragen werden.

Bei einer weiteren bevorzugten Ausführungsform wird als Ausgangsmaterial (Additiv) zur Oberflächenmodifizierung von Effektpigmenten basierend auf metallischen plättchenförmigen Substraten, die durch Nassvermahlung oder durch PVD-Verfahren hergestellt sein können, mit einer mittleren Substratdicke aus einem Bereich von 20 nm bis 90 nm wenigstens eine Fettsäure in einer Gesamtmenge aus einem Bereich von bevorzugt 4 Gew.-% bis 28 Gew.-%, weiter bevorzugt aus einem Bereich von 4 Gew.-% bis 25 Gew.-%, besonders bevorzugt aus einem Bereich von 6 Gew.-% bis 20 Gew.-% und ganz besonders bevorzugt aus einem Bereich von 8 Gew.-% bis 17 Gew.-%, jeweils bezogen auf das Gesamtgewicht des eingesetzten Effektpigments, eingesetzt.

Bei einer weiteren bevorzugten Ausführungsform wird als Ausgangsmaterial (Additiv) zur Oberflächenmodifizierung von Effektpigmenten basierend auf metallischen plättchenförmigen Substraten, die durch Nassvermahlung oder durch PVD-Verfahren hergestellt sein können, mit einer mittleren Substratdicke aus einem Bereich von 20 nm bis 90 nm wenigstens ein Silan in einer Gesamtmenge bevorzugt aus einem Bereich von 5 Gew.-% bis 39 Gew.-%, weiter bevorzugt aus einem Bereich von 10 Gew.-% bis 35 Gew.-%, besonders bevorzugt aus einem Bereich von 15 Gew.-% bis 32 Gew.-% und ganz besonders bevorzugt aus einem Bereich von 18 Gew.-% bis 30 Gew.-%, jeweils bezogen aus das Gesamtgewicht des eingesetzten Effektpigments, eingesetzt.

Bei ganz besonders bevorzugten Ausführungsformen werden wenigstens ein Phosphorsäureester und/oder wenigstens eine Phosphorsäure der Formel $(R\text{-}O)_n\text{-}P(O)(OR')(OR'')_m$, wobei jeweils R = linearer Alkylrest mit einer Kohlenstoffkette aus einem Bereich von $C_{14}$ bis $C_{18}$, und wobei R' und R'' unabhängig voneinander H, $CH_3$, $C_2H_5$ oder $C_3H_8$ sind und wobei n = 1 oder 2 und m = n - 1 und n + m = 2 ist, in einer Gesamtmenge aus einem Bereich von 14 Gew.-% bis 40 Gew.-%, bezogen auf das Gesamtgewicht des eingesetzten Effektpigments, als Ausgangsmaterial (Additiv) zur Oberflächenmodifizierung von Effektpigmenten basierend auf metallischen plättchenförmigen Substraten, die durch PVD-Verfahren hergestellt wurden mit einer mittleren Substratdicke $h_{50}$ aus einem Bereich von 15 nm bis 40 nm, bevorzugt 20 bis 40 nm verwendet. Weiter bevorzugt ist hierbei R' = R'' = H. Zudem ist besonders bevorzugt, dass n = 1 ist.

Bei ganz besonders bevorzugten Ausführungsformen werden wenigstens ein Phosphorsäureester eine Phosphorsäure der Formel $(R\text{-}O)_n\text{-}P(O)(OR')(OR'')_m$, wobei jeweils R = linearer Alkylrest mit einer Kohlenstoffkette aus einem Bereich von $C_{14}$ bis $C_{18}$, und wobei R' und R'' unabhängig voneinander H, $CH_3$, $C_2H_5$ oder $C_3H_8$ sind, und wobei n = 1 oder 2 und m = n - 1 und n + m = 2 ist, in einer Gesamtmenge aus einem Bereich von 14 Gew.-% bis 31 Gew.-%, bezogen auf das Gesamtgewicht des eingesetzten Effektpigments, als Ausgangsmaterial (Additiv) zur Oberflächenmodifizierung von Effektpigmenten basierend auf metallischen plättchenförmigen Substraten, die durch Nassvermahlung hergestellt wurden, mit einer mittleren Substratdicke aus einem Bereich von über 40 nm bis 90 nm, bevorzugt von 45 nm bis 80 nm verwendet. Weiter bevorzugt ist hierbei R' = R'' = H. Zudem ist besonders bevorzugt, dass n = 1 ist.

Bei weiter bevorzugten Ausführungsformen werden wenigstens eine Fettsäure R-COOH mit R = linearer Alkylrest mit einer Kohlenstoffkette aus einem Bereich von $C_{15}$ bis $C_{19}$ in einer Gesamtmenge aus einem Bereich von 11 Gew.-% bis 15 Gew.-%, bezogen auf das Gesamtgewicht des eingesetzten Effektpigments, als Ausgangsmaterial (Additiv) zur Oberflächenmodifizierung von Effektpigmenten basierend auf metallischen plättchenförmigen Substraten mit einer mittleren Substratdicke aus einem Bereich von 20 nm bis 90 nm und/oder wenigstens ein Silan $R\text{-}Si(OR')_3$ mit R = linearer Alkylrest mit einer Kohlenstoffkette aus einem Bereich von $C_{14}$ bis $C_{20}$ in einer Gesamtmenge aus einem Bereich von 21 Gew.-% bis 29 Gew.-%, bezogen auf das Gesamtgewicht des eingesetzten Effektpigments, zur Oberflächenmodifizierung von Effektpigmenten basierend auf metallischen plättchenförmigen Substraten mit einer mittleren Substratdicke aus einem Bereich von 20 nm bis 90 nm verwendet.

Unter "Gesamtmenge" wird erfindungsgemäß die komplette Menge an Ausgangsmaterial (Additiv) verstanden und zwar unabhängig davon, ob es sich ausschließlich um wenigstens einen Phosphorsäureester oder ausschließlich um wenigstens einen Phosphonsäureester oder um eine Mischung aus wenigstens einem Phosphorsäureester und wenigstens einem Phosphonsäureester oder um eine Mischung unterschiedlicher Phosphonsäuren oder um eine Mischung unterschiedlicher Fettsäuren oder um eine Mischung unterschiedlicher Silane handelt.

Die erfindungsgemäßen oberflächenmodifizierten Effektpigmente gemäß Anspruch 1 können in kosmetischen Formulierungen, insbesondere in Nagellackkompositionen, Verwendung finden. Die oberflächenmodifizierten Effektpigmente gemäß Anspruch 1 zeichnen sich insbesondere in Nagellackkompositionen durch ihr ausgezeichnetes Leafing-Verhalten aus.

**Erfindungsgemäße Nagellackkomposition:**

Die Erfindung richtet sich auf eine Nagellackkomposition, die den leafing-Effekt der Effektpigmente in ausgezeichneter Weise ermöglicht und erhält.

Diese erfindungsgemäße Nagellackkomposition enthält:

a) wenigstens ein mit einem Ausgangsmaterial (Additiv) oberflächenmodifiziertes Effektpigment wobei das Effektpigment ein plättchenförmiges Substrat und optional wenigstens eine auf dem Substrat aufgebrachte Beschichtung umfasst
b) wenigstens ein Kohlenwasserstoffharz als Bindemittel und
c) wenigstens ein Lösemittel oder Lösemittelgemisch,

wobei als Ausgangsmaterial (Additiv) zur Oberflächenmodifizierung des Effektpigments wenigstens eine Verbindung aus der Gruppe bestehend aus phosphorsäureesterhaltigen, phosphonsäureesterhaltigen, phosphonsäurehaltigen, fettsäurehaltigen und/oder silanhaltigen Verbindungen oder Mischungen hiervon, entnommen wird.

Die erfindungsgemäße Nagellackkomposition, welche wenigstens ein oberflächenmodifiziertes Effektpigment umfasst, weist im Unterschied zu den meisten kommerziell erhältlichen Nagellackkompositionen vorzugsweise keine Nitrocellulose auf. Das optische Erscheinungsbild der erfindungsgemäßen Nagellackkomposition wird, nach Applikation und Trocknung, maßgeblich durch das wenigstens eine oberflächenmodifizierte Effektpigment bestimmt.

Die erfindungsgemäße Nagellackkomposition umfasst das wenigstens eine oberflächenmodifizierte Effektpigment bevorzugt in einem Anteil aus einem Bereich von 0,3 Gew.-% bis 8,5 Gew.-%, weiter bevorzugt aus einem Bereich von 0,5 Gew.-% bis 5,0 Gew.-%, besonders bevorzugt aus einem Bereich von 0,6 Gew.-% bis 3,0 Gew.-% und ganz besonders bevorzugt aus einem Bereich von 0,8 Gew.-% bis 1,9 Gew.-%, jeweils bezogen auf das Gesamtgewicht der Nagellackkomposition.

Bei einer besonders bevorzugten Ausführungsform umfasst die erfindungsgemäße Nagellackkomposition wenigstens ein oberflächenmodifiziertes, optional beschichtetes, Aluminiumpigment, wobei das oberflächenmodifizierte Aluminiumpigment einen $D_{50}$-Wert aus einem Bereich von 5 $\mu$m bis 100 $\mu$m, bevorzugt aus einem Bereich von 2 $\mu$m bis 60 $\mu$m, aufweist und die mittlere Dicke des Aluminiumplättchens in einem Bereich von 10 nm bis 100 nm, bevorzugt in einem Bereich von 20 nm bis 40 nm, liegt. Bei dieser Ausführungsform wird als Ausgangsmaterial (Additiv) zur Oberflächenmodifizierung des optional beschichteten Aluminiumpigments bevorzugt wenigstens ein Phosphorsäureester R-O-P(O)(OR)(OR") mit R = linearer Alkylrest mit einer Kohlenstoffkette aus einem Bereich von $C_{14}$ bis $C_{18}$, R' = R" = H in einem Anteil aus einem Bereich von 10 Gew.-% bis 50 Gew.-%, bezogen auf das Gesamtgewicht des eingesetzten Effektpigments, eingesetzt. Die erfindungsgemäße Nagellackkomposition umfasst hierbei das vorstehend beschriebene oberflächenmodifizierte, optional beschichtete, Aluminiumpigment bevorzugt in einem Anteil aus einem Bereich von 0,6 Gew.-% bis 3,0 Gew.-%, besonders bevorzugt aus einem Bereich von 0,8 Gew.-% bis 1,9 Gew.-%, jeweils bezogen auf das Gesamtgewicht der Nagellackkomposition.

Unterhalb der bei den erfindungsgemäß oberflächenmodifizierten Effektpigmenten gemäß Anspruch 3 bzw. der verschiedenen, vorstehend beschriebenen Effektpigment-/Additivkombinationen für die jeweils für verschiedene Effektpigmenttypen angegebenen Mengen der als Ausgansmaterial (Additiv) zur Oberflächenmodifizierung zu verwendenden spezifischen Substanzen findet kein ausreichendes leafing der Effektpigmente statt. Oberhalb der jeweils angegebenen Mengen der Additive können gegebenenfalls zu große Mengen der Additive in die fertige Nagellackkomposition eingetragen werden.

Hierbei ist es besonders bevorzugt, dass die Effektpigmente in einem separaten Schritt mit dem Additiv beschichtet werden, bevor sie in das Nagellacksystem eingetragen werden.

Ein bevorzugtes Verfahren zur Herstellung der in dem erfindungsgemäßen Nagellack zu verwendenden oberflächenmodifizierten Effektpigmente umfasst daher die folgenden Schritte:

i. Suspendieren des optional mit wenigstens einem Metalloxid, Metallhydroxid und/oder Metalloxidhydrat beschichteten, metallischen plättchenförmigen Substrats oder des optional mit wenigstens einem Metalloxid, Metallhydroxid und/oder Metalloxidhydrat beschichteten nichtmetallischen plättchenförmigen Substrats in wenigstens einem Lösemittel,
ii. Zugabe des Phosphorsäurecetylesters oder des Phosphorsäurestearylesters bei optional erhöhter Temperatur zu der Suspension aus Schritt i. und Rühren der nun erhaltenen Suspension,
iii. Filtrieren, optional Trocknen, des gemäß Schritt ii. erhaltenen oberflächenmodifizierten Effektpigments.

Als Lösemittel wird bevorzugt ein mit dem erfindungsgemäßen Nagellack kompatibles Lösemittel verwendet. Besonders bevorzugt wird Butylacetat verwendet.

Bei PVD-Metallpigmenten oder bei besonders dünnen, durch Nassvermahlung hergestellten Metallpigmente wird man auf den Trocknungsschritt nach iii. verzichten und statt dessen diese Pigmente in einer Dispersion belassen.

Ein Teil der eingesetzten Additive wird nicht auf der Oberfläche des Effektpigments anhaften, da die Oberfläche bereits abgesättigt ist. Für eine hohe und gleichmäßige Bedeckung ist es jedoch wichtig, ausreichende Mengen an Additiv zur Verfügung zu stellen, da nur dann ein starker leafing-Effekt zu erwarten ist.

In den erfindungsgemäßen Nagellackkompositionen ordnen sich die oberflächenmodifizierten Effektpigmente bevorzugt an der Oberfläche des Klarlacks, also der erfindungsgemäßen Nagellackkomposition ohne oberflächenmodifizierte Effektpigmente, an. Unter "an der Oberfläche anordnen" wird erfindungsgemäß verstanden, dass sich die oberflächenmodifizierten Effektpigmente auf der Nagellackbasis und/oder ausgehend von der Grenzfläche Nagellackkomposition/Luft bzw. Nagellackkomposition/Überlackierung in Richtung des lackierten Untergrunds im an diese Grenzfläche anschließenden Drittel der Nagellackkomposition befinden. Bevorzugt schwimmen die oberflächenmodifizierten Effektpigmente im Klarlack auf und richten sich an der Klarlackoberfläche aus. Die oberflächenmodifizierten Effektpigmente zeichnen sich mithin in der erfindungsgemäßen Nagellackkomposition durch ein ausgeprägtes leafing-Verhalten aus.

Aufgrund dieses ausgeprägten leafing-Verhaltens der oberflächenmodifizierten Effektpigmente lassen sich erfindungsgemäß Nagellackkompositionen herstellen, welche ihr optisches Erscheinungsbild hauptsächlich dem der Nagellackbasis zugesetzten wenigstens einem oberflächenmodifizierten Effektpigment verdanken. In Abhängigkeit von dem wenigstens einem oberflächenmodifizierten Effektpigment sind so auf einfache Art und Weise Nagellackkompositionen zugänglich, welche sich nach Applikation und Trocknung durch ihren metallischen Charakter, ihren perlmuttartigen Schimmer, ihre Interferenzfarbe, einen Farbwechsel bei unterschiedlichem Betrachtungswinkel, intensive Glitzereffekte und/oder seidenmattes Aussehen auszeichnen, um nur einige erzielbare Effekte beispielhaft zu nennen. Selbstverständlich können der Nagellackkomposition je nach zu erzielendem optischen Effekt auch verschiedene oberflächenmodifizierte Effektpigmente zugesetzt werden, wobei hierbei sowohl die Oberflächenmodifizierung als auch die Effektpigmente verschieden voneinander sein können. Unter "voneinander verschiedenen Effektpigmenten" werden auch in ihrer Art identische Effektpigmente, beispielsweise Aluminiumpigmente, verstanden, die sich jedoch beispielsweise in ihrer Teilchengröße voneinander unterscheiden.

Als eine Besonderheit bei der Verwendung oberflächenmodifizierter Effektpigmente basierend auf Aluminiumplättchen oder basierend auf mit metallischem Silber beschichteten nichtmetallischen plättchenförmigen Substraten sei noch erwähnt, dass sich hiermit, nach Applikation und Trocknung Nagellackkompositionen mit Spiegelglanz erhalten lassen. Im Idealfall weisen die erfindungsgemäßen Nagellackkompositionen dann nach Applikation und Trocknung eine derart hohe Abbildschärfe auf, so dass ein Betrachter sich darin nahezu spiegeln kann.

Unter "Spiegelglanz" wird erfindungsgemäß verstanden, dass die Nagellackkomposition nach Applikation auf Glasplatten mittels einer Kastenrakel (Filmapplicator Erichsen System Wasag Model 288, Fa. Erichsen) in einer Nassfilmschichtdicke von 110 $\mu$m und nach anschließender Trocknung bei Raumtemperatur wenigstens 120 Glanzeinheiten, gemessen bei der 20° Geometrie (Byk-Gardner, Digitaler Katalog "Qualitätskontrolle für Lacke und Kunststoffe", Seite 16) und wenigstens 1100 Hazeeinheiten (Hlog) aufweist. Unter einem ausgezeichneten Spiegelglanz wird erfindungsgemäß verstanden, wenn wenigstens 150 Glanzeinheiten, gemessen bei der 20° Geometrie (Byk-Gardner, Digitaler Katalog "Qualitätskontrolle für Lacke und Kunststoffe", Seite 16) und wenigstens 1100 Hazeeinheiten (Hlog) erreicht werden. Die Glanz- und Hazeeinheiten (Hlog) werden hierbei vorzugsweise mit dem Messgerät haze-gloss, Fa. Byk-Gardner, bestimmt. Weisen die erfindungsgemäßen Nagellackapplikationen mikroskopisch kleine Strukturen auf, so wird hieran Licht nahe dem Reflexionswinkel gestreut, was die Abbildeschärfe der Nagellackapplikationen reduziert. Glanz (20° Geometrie) und Haze werden bei der Beurteilung des Spiegelglanzes vorzugsweise nicht unabhängig voneinander betrachtet. Je höher der Zahlenwert der Glanzeinheiten bei der 20° Geometrie ist, desto niedriger kann der Zahlenwert der Hazeeinheiten (Hlog) sein bzw. umgekehrt, je höher der Zahlenwert der Hazeeinheiten (Hlog) ist, desto niedriger kann der Zahlenwert der Glanzeinheiten bei der 20° Geometrie sein, um vom menschlichen Auge noch als Spiegelglanz wahrgenommen zu werden. Je höher der Zahlenwert der Glanzeinheiten bei der 20° Geometrie und je höher der Zahlenwert der Hazeeinheiten (Hlog) ist, desto höher ist die visuell wahrgenommene Abbildeschärfe der Nagellackapplikation.

Bei einer ganz bevorzugten Ausführungsform weisen die erfindungsgemäßen Nagellackkompositionen nach Applikation auf Glasplatten mittels einer Kastenrakel (Filmapplicator Erichsen System Wasag Model 288, Fa. Erichsen) in einer Nassfilmschichtdicke von 110 $\mu$m und nach anschließender Trocknung bei Raumtemperatur wenigstens 200 Glanzeinheiten, bevorzugt wenigstens 300 Glanzeinheiten, besonders bevorzugt wenigstens 400 Glanzeinheiten, ganz besonders bevorzugt wenigstens 450 bis 1500 Glanzeinheiten, jeweils gemessen bei der 20° Geometrie, und wenigstens 1200 Hazeeinheiten (Hlog), bevorzugt wenigstens 1300 Hazeeinheiten (Hlog), besonders bevorzugt wenigstens 1400 Hazeeinheiten (Hlog) und ganz besonders bevorzugt wenigstens 1410 bis 2000 Hazeeinheiten (Hlog) auf. Auch bei dieser Ausführungsform ist bei einem niedrigeren Zahlenwert der Glanzeinheiten, gemessen bei der 20° Geometrie, ein höherer Zahlenwert der Hazeeinheiten (Hlog) bevorzugt.

Bei einer bevorzugten Ausführungsform umfassen die erfindungsgemäßen Nagellackkompositionen, welche sich nach Applikation und Trocknung durch einen ausgezeichneten Spiegelglanz auszeichnen, 0,4 Gew.-% bis 2,7 Gew.-%, bevorzugt 0,5 Gew.-% bis 1,8 Gew.-%, jeweils bezogen auf das Gesamtgewicht der Nagellackkomposition, oberflächenmodifizierte Effektpigmente basierend auf Aluminiumplättchen, wobei die Aluminiumplättchen einen $D_{50}$-Wert aus einem Bereich von 2 $\mu$m bis 500 $\mu$m, bevorzugt aus einem Bereich von 2,5 $\mu$m bis 90 $\mu$m, und eine mittlere Dicke aus einem Bereich von 15 nm bis 1000 nm, bevorzugt aus einem Bereich von 18 nm bis 60 nm, aufweisen.

Bei einer besonders bevorzugten Ausführungsform umfassen die erfindungsgemäßen Nagellackkompositionen, welche sich nach Applikation durch einen ausgezeichneten Spiegelglanz auszeichnen, 0,6 Gew.-% bis 4,9 Gew.-%, bevorzugt 0,3 Gew.-% bis 3,8 Gew.-%, jeweils bezogen auf das Gesamtgewicht der Nagellackkomposition, oberflächenmodifizierte Effektpigmente basierend auf Aluminiumplättchen, wobei die Aluminiumplättchen einen Dso-Wert aus einem Bereich von 5 $\mu$m bis 150 $\mu$m, bevorzugt aus einem Bereich von 10 $\mu$m bis 60 $\mu$m, und eine mittlere Dicke aus einem Bereich von 10 nm bis 600 nm, bevorzugt aus einem Bereich von 20 nm bis 100 nm, aufweisen.

Bei einer bevorzugten Ausführungsform weisen die erfindungsgemäßen Nagellackkompositionen nach Applikation auf einer Glasplatte mittels einer Kastenrakel (Filmapplicator Erichsen System Wasag Model 288, Fa. Erichsen) in einer Nassfilmschichtdicke von 100 $\mu$m und anschließender Trocknung bei Raumtemperatur wenigstens 200 Glanzeinheiten, gemessen bei der 20° Geometrie, und wenigstens 1200 Hazeeinheiten (Hlog) auf.

Bei einer bevorzugten Ausführungsform umfasst die erfindungsgemäße Nagellackkomposition wenigstens ein oberflächenmodifiziertes Effektpigment in einem Anteil aus einem Bereich von 0,1 Gew.-% bis 8,4 Gew.-%, bevorzugt in einem Anteil aus einem Bereich von 0,15 Gew.-% bis 6,9 Gew.-%, besonders bevorzugt in einem Anteil aus einem Bereich von 0,2 Gew.-% bis 4,3 Gew.-%, jeweils bezogen auf das Gesamtgewicht der Nagellackkomposition, wenigstens ein kohlenwasserstoffhaltiges Harz und wenigstens ein Lösemittel.

Bei einer weiteren bevorzugten Ausführungsform umfasst die erfindungsgemäße Nagellackkomposition wenigstens ein oberflächenmodifiziertes Effektpigment basierend auf einem metallischen plättchenförmigen Substrat, wobei das Effektpigment eine mittlere Teilchengröße $D_{50}$ aus einem Bereich von 2 μm bis 60 μm und eine mittlere Gesamtdicke aus einem Bereich von 10 nm bis 150 nm aufweist.

Bindemittel:

Die erfindungsgemäßen Nagellackkompositionen umfassen als Bindemittel wenigstens ein Kohlenwasserstoffharz, wobei das Bindemittel bevorzugt einen Bindemittelfestkörperanteil aus einem Bereich von 25 Gew.-% bis 64 Gew.-%, weiter bevorzugt aus einem Bereich von 25 Gew.-% bis 60 Gew.-%, weiter bevorzugt aus einem Bereich von 28 Gew.-% bis 55 Gew.-%, besonders bevorzugt aus einem Bereich von 29 Gew.-% bis 50 Gew.-% und ganz besonders bevorzugt aus einem Bereich von 35 Gew.-% bis 43 Gew.-%, jeweils bezogen auf das Gesamtgewicht der Nagellackkomposition, aufweist.

Unterhalb eines Bindemittelanteils von 25 Gew.-% waren keine guten optischen Effekte durch die Effektpigmente im applizierten Nagellack mehr zu erkennen.

Oberhalb von 60 Gew.-% lässt die optische Qualität der Effektpigmente ebenfalls nach und zunehmend wird die Viskosität der erfindungsgemäßen Nagellackkompositionen zu hoch.

Die erfindungsgemäßen Nagellackkompositionen umfassen vorzugsweise als Bindemittel Kohlenwasserstoffharze mit einem mittleren Molekulargewicht ($M_w$) aus einem Bereich von 800 bis 6000, bevorzugt aus einem Bereich 900 bis 5000, oder aus einem Bereich von 8000 bis 10000, bevorzugt aus einem Bereich von 8500 bis 9300. Das mittlere Molekulargewicht Mw wurde mittels Gel-Permeations-Chromatographie (GPC) mit einem Polystyrol Standard bestimmt.

Unter Kohlenwasserstoffharze versteht man synthetische Harze, die durch Reaktion von Kohlenwasserstoffen (außer Olefinen) mit sich selbst in Gegenwart von Aluminiumchlorid oder Schwefelsäure als Katalysator entstehen. Die Kohlenwasserstoffharze werden entsprechend ihres Aufbaues in Petrolharze, Terpenharze und Cumaron-Inden-Harze unterteilt. Zu den Kohlenwasserstoffharze werden auch die Reaktionsprodukte aus Xylol und Formaldehyd, die Xylol-Formaldehyd-Harze gerechnet.

Die Herstellung der Kohlenwasserstoffharze erfolgt in bekannter Weise durch Erhitzen von hochsiedenden Fraktionen der Benzinpyrolyse (Pyrolyseöl) oder der isoprenfreien $C_5$-Fraktion der Benzinpyrolyse in Anwesenheit von Aluminiumchlorid. Die Kohlenwasserstoffharze sind in den meisten organischen Lösungsmitteln, z. B. Ester, Ether, Chlorkohlenwasserstoffe und Aromaten, löslich.

Ohne an eine Theorie gebunden zu sein, vermuten die Erfinder, dass bei der Verwendung von polaren Bindemitteln die mit geeigneten Additiven beschichteten Effektpigmente immer noch teilweise von dem Bindemittel benetzt werden und daher nicht den gewünschten leafing- Effekt aufweisen. Hingegen sind die Harze der erfindungsgemäßen Nagellackkomposition sehr unpolar und benetzen demzufolge die Effektpigmente nicht. Dadurch können die Effektpigmente den leafing-Effekt besser ausbilden.

In bevorzugten Ausführungsformen werden Nagellackkompositionen enthaltend aromatische Kohlenwasserstoffharze verwendet.

Die erfindungsgemäßen Nagellackkompositionen können als Bindemittel Kohlenwasserstoffharze, wie beispielsweise Kristalex F100 Hydrocarbon Resin, Kristalex 5140 Hydrocarbon Resin, Kristalex 3070 Hydrocarbon Resin, Kristalex 3085 Hydrocarbon Resin, Kristalex F115 Hydrocarbon Resin, jeweils Fa. Eastman umfassen.

Bevorzugt umfassen die erfindungsgemäßen Nagellackkompositionen als Bindemittel die Kohlenwasserstoffharze Kristalex F100 Hydrocarbon Resin und Kristalex 5140 Hydrocarbon Resin.

In besonders bevorzugten Ausführungsformen enthält die erfindungsgemäße Nagellackkomposition kohlenwasserstoffhaltiges Harz in einer Menge, die 80 bis 100 Gew.-%, weiter bevorzugt 90 bis 99 Gew.-% des gesamten organischen Bindemittels ausmacht.

Die Verwendung von Kohlenwasserstoffharzen in Nagellacken als Hauptbestandteil des Bindemittels ist nach den Kenntnissen der Erfinder nicht üblich. Gewöhnlicherweise sind Kohlenwasserstoffharze sehr selten Bestandteile von Nagellacken und wenn, so werden sie mit anderen Bindemitteln in vergleichsweise eher geringen Anteilen eingesetzt.

Bei einer besonders bevorzugten Ausführungsform umfassen die erfindungsgemäßen Nagellackkompositionen wenigstens zwei voneinander verschiedene Kohlenwasserstoffharze mit einem ersten mittleren Molekulargewicht Mw von 1200 bis 1600 g/mol und einem zweiten mittleren Molekulargewicht Mw 4500 bis 5500 g/mol im Gewichtsverhältnis 1:1 bis 1:10, bevorzugt 1:1 bis 1:8, besonders bevorzugt 1:1 bis 1:4 und ganz besonders bevorzugt 1:1 bis 1:2 der beiden verschiedenen Kohlenwasserstoffharze.

In weitere Ausführungsformen enthält die Nagellackkomposition keine oder nahezu keine zusätzlichen Bindemittel der Gruppe aus Nitrocellulose, Polyesterharze, Polyvinylharze, Alkydharz, Epoxidharze oder Celluloseacetatbutyrat. Diese Bindemittel sind bevorzugt in Mengenanteilen von unter 10 Gew.-%, weiter bevorzugt unter 5 Gew.-% und besonders bevorzugt von unter 1 Gew.-% und ganz besonders bevorzugt unter 0,1 Gew.-%, jeweils bezogen auf das Gesamtgewicht der Kohlenwasserstoffharze sowie zusätzliche Bindemittel, enthalten. Diese Bindemittel haben sich eher als hinderlich zur Erzielung eines wirklich starken Spiegeleffekts erwiesen.

Ohne an eine Theorie gebunden zu sein vermuten die Erfinder, dass bei Nagellackkompositionen die die oben genannten Bindemittel enthalten, diese aufgrund ihrer stärkeren Polarität zumindest teilweise die Effektpigmente benetzen und diese dadurch schlechtere leafing-Eigenschaften aufweisen.

Lösemittel:

Die erfindungsgemäßen Nagellackkompositionen enthalten bevorzugt bestimmte Lösemittel. Als Lösungsmittel können den erfindungsgemäßen Nagellackkompositionen Ethylacetat, Butylacetat, Isopropanol zugesetzt werden.

Bevorzugt enthält die erfindungsgemäße Nagellackkomposition als Lösemittel eine Mischung aus Isopropanol, Ethylacetat und Butylacetat.

Besonders bevorzugt enthält die erfindungsgemäße Nagellackkomposition die Lösemittelmischung aus Isopropanol, Ethylacetat und Butylacetat in einer Menge, die 70 bis 100 Gew.-%, weiter bevorzugt 75 bis 98 Gew.-%, bezogen auf das gesamte Lösemittel der Nagellackkomposition.

Hierbei ist es unerheblich, ob diese bevorzugten Lösemittel über die Bindemittel oder die Effektpigmentdispersion eingetragen werden.

In weiteren bevorzugten Ausführungsformen beträgt bei dieser Lösemittelmischung das Verhältnis von Butylacetat zur Lösemittelmischung 50 bis 99 Gew.-% und besonders bevorzugt 55 bis 98,5 Gew.-%.

Bei einer weiteren besonders bevorzugten Ausführungsform liegt der Anteil von Isopropanol unter 20 Gew.-%, vorzugsweise unter 15 Gew.-%, und weiter bevorzugt unter 10 Gew.-%, jeweils bezogen auf das gesamte Lösemittel. Zu hohe Anteile an Isopropanol in der erfindungsgemäßen Nagellackkomposition führen zu einem schlechten optischen Erscheinungsbild der Effektpigmente. Vermutlich ist dies auf eine zu schnelle Trocknung der Nagellacke nach ihrer Applikation zurückzuführen.

Bei einer weiteren besonders bevorzugten Ausführungsform umfasst die erfindungsgemäße Nagellackkomposition wenigstens ein oberflächenmodifiziertes Effektpigment basierend auf einem metallischen plättchenförmigen Substrat, wenigstens zwei voneinander verschiedene Kohlenwasserstoffharze sowie die Lösungsmittel Ethylacetat, Butylacetat, Isopropanol.

Die erfindungsgemäßen Nagellackkompositionen sind äußerst einfach auf einem menschlichen oder künstlichen Finger- und/oder Fußnagel applizierbar. Sie zeichnen sich während der Applikation durch einen guten Verlauf aus und bilden nach der anschließenden Trocknung einen homogenen Film auf einem menschlichen oder künstlichen Finger- und/oder Fußnagel.

In bevorzugten Ausführungsformen enthält der erfindungsgemäße Nagellack 50 Gew.-% bis 70 Gew.-%, bevorzugt 55 Gew.-% bis 68 und besonders bevorzugt 57 bis 65 Gew.-%, jeweils bezogen auf das Gewicht des gesamten Nagellacks.

Unterhalb von 55 Gew.-% steigt die Viskosität des Nagellacks zu stark an und die Effektpigmente können sich nicht optimal orientieren, was zu einer Verminderung bis Verlust des Spiegelglanzes führt.

Oberhalb von 70 Gew.-% verringert sich die Viskosität des Nagellacks, was zu einem schlecht kontrollierbaren Auftrag des Nagellacks auf den Fingernagel führt.

Weitere Bestandteile:

Die erfindungsgemäßen Nagellackkompositionen können zusätzlich einen oder mehrere weitere Bestandteile enthalten. Hier sind insbesondere Weichmacher und Antioxidantien zu nennen.

Als Weichmacher können beispielsweise Glycole und ihre Derivate wie beispielsweise Diethylen- glycolethylether, Diethylenglycolmethylether, Diethyleneglycolbutylether oder weiterhin Diethylenglycolhexylether, Ethylenglycolethylether, Ethylenglycolmethylether, Ethyleneglycolbutylether, Ethylenglycolhexylether, Glycolesters, Derivate von Propylenglycol und insbesondere Propylenglycolphenylether, Propylenglycoldiacetate, Dipropylenglycolbutylether, Tripropylenglycolbutylether, Propylenglycolmethylether, Dipropylenglycolethylether, Tripropylenglycolmethylether und Diethylenglycolmethylether, Propylenglycobutylether, oder Mischungen hiervon, verwendet werden.

Weiterhin können als Weichmacher insbesondere Ester von Carbonsäuren, wie beispielsweise von Citraten, insbesondere von Trimethylcitrat, Tributylcitrat, Trimethylacetylcitrat, Tributylacetylcitrat, Triethyl-2-hexylacetylcitrate oder von Phthalatesn, insbesondere Dimethoxyethylphthalat; oder von Phosphaten, insbesonmdere von Tricresylphosphat, Tributylphosphat, Triphenylphosphat, Tributoxyethylphosphate oder von Tartraten, insbesondere Dibuty tartrat; Adipaten, Carbonaten, Sebacaten; Benzylbenzoat, Butylacetylricinoleat, Glycerylacetylricinoleat, Butylglycolat, Kampher, Glyceroltriacetate, N-ethyl-o,p-toluolsulfonamid, Oxyethylen Verbindungen wie beispielsweise Oxyethylenöle, insbesondere pflanzliche Öle, wie beispielsweise Castoröl, Kohlenwasserstofföle und Mischungen hiervon.

Bevorzugte Weichmacher sind insbesondere Kohlenwasserstofföle.

Die Gewichtsanteile der Weichmacher an der gesamten Nagellackkomposition betragen bevorzugt einen Bereich von 0 bis 15 Gew.-%, weiter bevorzugt von 1 bis 10 Gew.-%, und besonders bevorzugt von 5 bis 10 Gew.-%.

Antioxidantien:

Die erfindungsgemäße Nagellackkomposition kann weiterhin ein oder mehrere Antioxidantien enthalten.

Unter "Antioxidantien" werden Verbindungen verstanden, die die Bestandteile des erfindungsgemäßen Nagellacks, insbesondere die Kohlenwasserstoffbindemittel, vor dem Einfluss von Sauerstoff, Hitze, Ozon, und/oder UV-Strahlung schützen. Es können eine oder mehrere derartige Verbindungen verwendet werden.

Beispiele für derartige Verbindungen sind IRGANOX® 1010, IRGANOX® 565, IRGANOX® 1076 (Fa. BASF) oder Schwefel-haltige Antioxidantien wie beispielsweise Zinkdibutyldithiocarbamat (PERKACIT ZDBC.(Fa. Performance additives Italy S.p.A). Bevorzugt werden die Antioxidantien in Mengen aus einem Bereich von 0 bis 5 Gew-%, weiter bevorzugt aus einem Bereich von 0,05 bis 1 Gew.-%, bezogen auf die gesamte Nagellackkomposition, eingesetzt.

Weitere Additive:

Die erfindungsgemäße Nagellackkomposition kann weiterhin übliche weitere Additive enthalten, wie sie dem Fachmann bekannt sind.

Derartige weitere Additive sind beispielsweise Antiabsetzmittel, Konservierungsstoffe, Öle, Wachse, Radikalfänger, Benetzungsadditive, Dispergierhilfsmittel, Benetzungshilfsmittel, Antischaummittel, Parfüm, Neutralisiermittel, Verdicker, UV-Blocker Feuchthaltemittel, Vitamine, Proteine und Mischungen hiervon.

In weiteren Ausführungsformen enthält die erfindungsgemäße Nagellackkomposition vorzugsweise keine Antiabsetzmittel. Etwaig abgesetztes oberflächenmodifiziertes Effektpigmente lassen sich erstaunlicherweise in aller Regel auch ohne den Zusatz von Antiabsetzmitteln durch einfaches Aufschütteln wieder dispergieren.

Die erfindungsgemäße Nagellackkomposition weist vorzugsweise eine Viskosität von 10 sec bis 16 sec auf, gemessen mit einem DIN Auslaufbecher (DIN 4 mm) gemäß DIN 53211.

In bevorzugten Ausführungsformen enthält die erfindungsgemäße Nagellackkomposition Aluminium-PVD Effektpigmente mit einer mittleren Dicke $h_{50}$ aus einem Bereich von 14 bis 40 nm, bevorzugt aus einem Bereich von 15 bis 35 nm, die mit Phosphorsäurecetylester oder Laurylphosphonsäure als leafing-Additiv beschichtet sind, aromatische Kohlenwasserstoffharze als Bindemittel und eine Mischung aus Isopropanol, Ethylacetat und Butylacetat als Lösemittel, wobei diese Lösemittelmischung 70 bis 100 Gew.-% des gesamten Lösemittels der Nagellackkomposition ausmachen.

Bei einer weiteren bevorzugten Ausführungsform enthält die erfindungsgemäße Nagellackkomposition Aluminium-PVD Effektpigmente mit einer mittleren Dicke $h_{50}$ aus einem Bereich von 14 bis 40 nm, bevorzugt aus einem Bereich von 15 bis 35 nm, sowie wenigstens zwei voneinander verschiedene aromatische Kohlenwasserstoffharze und eine Mischung aus Isopropanol, Ethylacetat und Butylacetat als Lösemittel, wobei diese Lösemittelmischung 70 bis 100 Gew.-% des gesamten Lösemittels der Nagellackkomposition ausmachen. Bevorzugt werden auch hier als leafing-Additiv Phosphorsäurecetylester oder Laurylphosphonsäure verwendet.

Gegenstand der Erfindung ist weiterhin ein Verfahren zur Herstellung der erfindungsgemäßen Nagellackkomposition, umfassend die Schritte

    i) Oberflächenmodifizierung des Effektpigments durch ein Additiv in einer Dispersion in einem Lösemittel,
    ii) Lösen des Kohlenwasserstoffharzes in einem Lösemittel oder Lösemittelgemisch
    iii) Vermischen und Homogenisieren der Dispersion nach i) mit der Bindemittellösung nach ii).

Bevorzugt wird hierbei der Schritt i) ausgeführt nach folgendem Verfahren zur Herstellung der oberflächenmodifizierten Effektpigmente umfassend die folgenden Schritte:

    i. Suspendieren des optional mit wenigstens einem Metalloxid, Metallhydroxid und/oder Metalloxidhydrat beschichteten, metallischen plättchenförmigen Substrats oder des optional mit wenigstens einem Metalloxid, Metallhydroxid und/oder Metalloxidhydrat beschichteten nichtmetallischen plättchenförmigen Substrats in wenigstens einem Lösemittel,
    ii. Zugabe des Phosphorsäurecetylesters oder des Phosphorsäurestearylesters bei optional erhöhter Temperatur zu der Suspension aus Schritt i. und Rühren der nun erhaltenen Suspension,
    iii. Filtrieren, optional Trocknen, des gemäß Schritt ii. erhaltenen oberflächenmodifizierten Effektpigments.

Das Lösen des Kohlenwasserstoffharzes in einem Lösemittel erfolgt bevorzugt in einem Gemisch aus mindestens zwei, bevorzugt drei Lösemitteln. Besonders bevorzugt wird eine Mischung aus Isopropanol, Ethylacetat und Butylglycol verwendet.

In weiteren bevorzugten Ausführungsformen wird das Lösemittel des Schrittes i) ebenfalls aus Isopropanol, Ethylacetat und Butylglycol oder einer Mischung hiervon bestehen, um nicht weitere, möglicherweise störende Lösemittel in den Nagellack einzutragen.

Weiterhin ist es bevorzugt, bei der Auswahl der metallischen Effektpigmente solche zu wählen, die in einer Paste oder einer Dispersion der bevorzugten Lösemittel Isopropanol, Ethylacetat und Butylglycol vorliegen, da das Lösemittel der Metalleffektpigmentpaste ebenfalls in geringen Mengen in den erfindungsgemäßen Nagellack gelangt, falls nicht aufwendige Umnetzungsschritte eingesetzt werden.

Gegenstand der Erfindung ist ebenfalls ein Verfahren zur Beschichtung eines natürlichen oder künstlichen Fingernagels umfassend die Schritte:

> a) Beschichtung des natürlichen oder künstlichen Fingernagels mit einer erfindungsgemäßen Nagellackkomposition und anschließendem Trocknen des Nagellacks,
> b) optional darauffolgende Beschichtung des Nagellacks mit einem Klarlack.

Das Aufbringen des Klarlacks erhöht beträchtlich die Abriebbeständigkeit der Nagellackierung. Aufgrund des ausgeprägten leafing-Effektes der Effektpigmente im Nagellack weist dieser naturgemäß eine eher geringe Abriebbeständigkeit auf.

Vor dem Schritt a) kann ebenfalls schon eine Beschichtung des natürlichen oder künstlichen Fingernagels mit einem Klarlack erfolgen, um eine möglichst ebene Oberfläche herzustellen. Diese Vorgehensweise empfiehlt sich, falls die Findernägel eine hohe Rauigkeit aufweisen.

Die nachfolgende Beschichtung mit Klarlack in Schritt b) kann mit dem gleichen oder einem anderen Klarlack wie der erfindungsgemäße Nagellack durchgeführt werden. Allerdings enthält dieser Klarlack auf keinen Fall Effektpigmente, da diese den gewünschten Effekt des erfindungsgemäßen Nagellacks überlagern würden.

Allerdings kann der Klarlack in Schritt b) konventionelle Farbpigmente oder Farbstoffe enthalten. Gerade in Kombination mit metallischen PVD-Aluminiumpigmenten oder mit dünnen, durch Nassvermahlung hergestellten Aluminiumeffektpigmenten mit einem $h_{50}$-Wert von 20 bis unter 100 nm lassen sich optisch sehr reizvolle Effekte realisieren. Bevorzugt weisen hierbei die mit den Effektpigmenten pigmentierte erfindungsgemäße Nagellackkompositionen nach Schritt a) einen Spiegelglanz auf.

Bei einer weiteren Ausführungsform kann die erfindungsgemäße Nagellackkomposition mit einem niedrig viskosen UV härtenden Klarlack überlackiert werden um die Abriebbeständigkeit der erfindungsgemäßen Nagellackkomposition zu erhöhen.

Bevorzugt können auch lösemittelbasierende Klarlacke verwendet werden. Ohne an eine Theorie gebunden zu sein, basiert der Klarlack bevorzugt auf polaren Bindemitteln, die nur gering mit den Kohlenwasserstoffharzen des erfindungsgemäßen Klarlacks wechselwirken. In besonders bevorzugten Ausführungsformen basierend diese Klarlacke auf Bindemitteln wie Polyvinyl Butyral (PVB), Polyvinylpyrrolidon (PVP) oder Mischungen hiervon.

Weiterhin enthält der Klarlack bevorzugt Lösemittel, die nicht die unpolaren Kohlenwasserstoffharze des erfindungsgemäßen Lacks lösen bzw. anlösen. Beispielsweise kann hierfür Isopropanol verwendet werden. Andernfalls können auch die leafing-Effektpigmente wieder angelöst und in ihrer Orientierung gestört werden, wodurch der Spiegelglanzeffekt gestört wird.

Überraschenderweise weisen die erfindungsgemäßen Klarlacke eine sehr gute Haftungsbeständigkeit auf dem erfindungsgemäßen Nagellack auf.

**Beispiele**

Die nachfolgenden Beispiele dienen der näheren Beschreibung der Erfindung und sollen in keinerlei Hinsicht einschränkend sein. Alle Prozentangaben sind Gewichtsprozentangaben. Die Begriffe NFA (nichtflüchtiger Anteil), Festkörperanteil und Feststoffgehalt sind austauschbar verwendbar.

**I Herstellung der erfindungsgemäßen, bevorzugt in einer Nagellackkomposition zu verwendenden, oberflächenmodifizierten Effektpigmente und Herstellung der oberflächenmodifizierten Effektpigmente gemäß Anspruch 1**

Beispiele 1 bis 7:

In einem 1 L-Doppelwandreaktor wurden 300 g des Aluminiumeffektpigments METALURE A 41010 AE (Dispersion in Essigsäureethylester, Feststoffgehalt 10%, $D_{50}$ (Horiba LA-930) = 9,5 $\mu$m bis 10,5 $\mu$m, Fa. ECKART GmbH) in einem Lösemittel gemäß nachstehender Tabelle 2 bei 200 rpm/min dispergiert und auf 40°C erhitzt. Anschließend wurde das Additiv Phosphorsäurecetylester (CAS Nummer: 3539-43-3, Hostaphat CC 100, Fa. Clariant) gemäß nachstehender Tabelle 2, gelöst in 30 g des zum Dispergieren verwendeten Lösemittels, zur Aluminiumeffektpigment-Dispersion zugegeben. Nach 6 Stunden rühren bei 90°C wurde die Mischung abgekühlt und über einen Büchnertrichter filtriert. Es wurden oberflächenmodifizierte Aluminiumeffektpigmente als 5-25%ige Dispersionen erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergaben.

Tabelle 2:

| Beispiel | Lösemittel | Menge Lösemittel [g] | Menge Additiv [g] | NFA [%][1] |
|---|---|---|---|---|
| 1 | Butylacetat 85/100 | 0 | 3 | 10 |
| 2 | Butylacetat 85/100 | 50 | 3 | 9 |
| 3 | Butylacetat 85/100 | 100 | 3 | 11 |
| 4 | Butylacetat 85/100 | 200 | 3 | 7 |
| 5 | Butylacetat 85/100 | 300 | 3 | 23 |
| 6 | Butylacetat 85/100 | 300 | 6 | 21 |
| 7 | Ethylacetat | 200 | 5,4 | 6 |
| [1] Nichtflüchtiger Anteil des oberflächenmoditizierten Effektpigments. | | | | |

Beispiele 8 bis 12:

In einem 1L-Doppelwandreaktor wurden 300 g des Aluminiumeffektpigments METALURE A 41506 EN (Dispersion in Ethanol, Feststoffgehalt 15%, $D_{50}$ (Horiba LA-930) = 5,5 $\mu$m bis 6,5 $\mu$m, Fa. ECKART GmbH) in 300 g Lösemittel gemäß nachstehender Tabelle 3 bei 200 rpm/min dispergiert und auf 40°C erhitzt. Anschließend wurde das Additiv gemäß nachstehender Tabelle 3 in 30 g des entsprechenden Lösemittels zur Aluminiumeffektpigment-Dispersion zugegeben. Nach 6 Stunden rühren bei 90°C wurde die Mischung abgekühlt und über einen Büchnertrichter filtriert. Es wurden jeweils oberflächenmodifizierte Aluminiumeffektpigmente als 10-20%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergaben.

Tabelle 3

| Beispiel | Lösemittel | Additiv | Menge [g] | NFA [%] |
|---|---|---|---|---|
| 8 | Butylacetat 85/100 | Hostaphat CC 100[2] | 3 | 18 |
| 9 | Butylacetat 85/100 | Hostaphat CC 100 | 6 | 15 |
| 10 | Methoxypropanol | Hostaphat CC 100 | 9 | 18 |
| 11 | Methoxypropanol | Laurylphosphonsäure[3] | 6 | 14 |
| 12 | Butylacetat 85/100 | Laurylphosphonsäure | 6 | 15 |
| [2] Phosphorsäurecetylester, CAS Nummer: 3539-43-3, Fa. Clariant. [3] Fa. Rhodia. | | | | |

Beispiel 13 :

In einem 1L-Doppelwandreaktor wurden 300 g des Aluminiumeffektpigments METALURE A 41010 AE (Dispersion in Essigsäureethylester, Feststoffgehalt 10%, $D_{50}$ (Horiba LA-930) = 9,5 $\mu$m bis 10,5 $\mu$m, Fa. ECKART GmbH) in 50 g Butylacetat 85/100 bei 200 rpm/min dispergiert und auf 80°C erhitzt. Anschließend wurden 3 g des Additivs Phosphorsäurecetylester (CAS Nummer: 3539-43-3, Hostaphat CC 100, Fa. Clariant) in 30 g Butylacetat 85/100 zur Aluminiumeffektpigment-Dispersion zugegeben. Nach 6 Stunden rühren bei 40°C wurde die Mischung abgekühlt und über einen Büchnertrichter filtriert. Es wurde ein oberflächenmodifiziertes Aluminiumeffektpigment als 15%ige Dispersion erhalten, welches nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergab.

Beispiel 14:

In einem 1L-Doppelwandreaktor wurden 300 g des Aluminiumeffektpigments METALURE A 41010 AE (Dispersion in Essigsäureethylester, Feststoffgehalt 10%, $D_{50}$ (Horiba LA-930) = 9,5 $\mu$m bis 10,5 $\mu$m, Fa. ECKART GmbH) in 50 g Butylacetat 85/100 bei 200 rpm/min dispergiert und auf 60°C erhitzt. Anschließend wurden 3 g des Additives Phosphorsäurecetylester (CAS Nummer: 3539-43-3, Hostaphat CC 100, Fa. Clariant) in 30 g Butylacetat 85/100 zur Aluminiumeffetkpigment-Dispersion zugegeben. Nach 6 Stunden rühren bei 40°C wurde die Mischung abgekühlt und über einen Büchnertrichter filtriert. Es wurde ein oberflächenmodifiziertes Aluminiumeffektpigment als 10%ige Dispersion erhalten, welches nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergab.

Beispiele 15 bis 18:

In einem 1L-Doppelwandreaktor wurden 300 g Effektpigment gemäß nachstehender Tabelle 4 in 300 g Lösemittel gemäß nachstehender Tabelle 4 bei 200 rpm/min dispergiert und auf 90°C erhitzt. Anschließend wurde das Additiv Phosphorsäurecetylester (CAS Nummer: 3539-43-3, Hostaphat CC 100, Fa. Clariant) in 30 g des entsprechenden Lösemittels zur Effektpigment-Dispersion zugegeben. Nach 6 Stunden rühren bei 40°C wurde die Mischung abgekühlt und über einen Büchnertrichter filtriert. Es wurden jeweils oberflächenmodifizierte Effektpigmente als Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack ergaben, dessen optisches Erscheinungsbild auf das jeweils eingesetzte Effektpigment zurückzuführen war.

Tabelle 4

| Beispiel | Effektpigment | Additiv [g] | Lösemittel | NFA [%] |
|---|---|---|---|---|
| | | | | |
| 15 | SILVERSHINE S 1500[4] | 3 | Monopropylenglykolmonomethylether[8] | 35 |
| 17 | SYNCRYSTAL Silk Blue[5] | 3 | Monopropylenglykolmonomethylether | 72 |
| 17 | METALURE A 31017 AE[6] | 3 | Butylacetat 85/100 | 12 |
| 18 | METALURE A 31017 AE[6] | 6 | Butylacetat 85/100 | 12 |

[4] Aluminiumeffektpigmentpaste, Feststoffgehalt 23 bis 27 %, $D_{50}$ (CILAS 1064) = 12 $\mu$m bis 18 $\mu$m, Fa. ECKART GmbH.

[5] Titandioxidbeschichtetes Perlglanzpigment mit blauer Interferenzfarbe, Dso (Malvern Mastersizer 2000) = 13 $\mu$m, Fa. ECKART GmbH.[67] Aluminiumeffektpigment, Dispersion in Essigsäureethylester, Feststoffgehalt 10%, Dso (Horiba LA-930) = 17 $\mu$m, Fa. ECKART GmbH.

Beispiele 19 bis 24:

In einem 1L-Doppelwandreaktor wurden 300 g des Aluminiumeffektpigments METALURE L 55350 AE (Dispersion in Essigsäureethylester, Feststoffgehalt 10%, $D_{50}$ (Horiba LA-930) = 11 $\mu$m bis 12 $\mu$m, Fa. ECKART GmbH) in 300 g Lösemittel gemäß nachstehender Tabelle 5 bei 200 rpm/min dispergiert und auf 40°C erhitzt. Anschließend wurde das jeweils verwendete Additiv in 30 g Butylacetat 85/100 zur Aluminiumeffektpigment-Dispersion zugegeben. Nach 6 Stunden rühren bei 100°C wurde die Mischung abgekühlt und über einen Büchnertrichter filtriert. Es wurden oberflächenmodifizierte Aluminiumeffektpigmente jeweils als 11-20%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack mit spiegelähnlichem Glanz ergaben.

Tabelle 5

| Beispiel | Additiv | Lösemittel | Menge [g] | NFA [%] |
|---|---|---|---|---|
| 19 | Laurylphosphonsäure | Butylacetat 85/100 | 3 | 18 |
| 20 | Laurylphosphonsäure | Butylacetat 85/100 | 6 | 18 |
| 21 | Laurylphosphonsäure | Monopropylenglykolmonomethylether | 6 | 18,5 |

(fortgesetzt)

| Beispiel | Additiv | Lösemittel | Menge [g] | NFA [%] |
|---|---|---|---|---|
| 22 | Hostaphat CS 120[6] | Butylacetat 85/100 | 6 | 15 |
| 23 | Hostaphat CS 120 | Butylacetat 85/100 | 15 | 15 |
| 24 | Hostaphat CC 100 | Butylacetat 85/100 | 3 | 11,7 |
| [6] Phosphorsäurestearylester, CAS-Nummer: 39471-52-8, Fa. Clariant. | | | | |

Beispiele 25 bis 31, Vergleichsbeispiel 1:

In einem 1L-Doppelwandreaktor wurden 200 g Silverdream Moonlight 50IL(Feststoffgehalt 50%, $D_{50}$ (CILAS 1064) = 15 $\mu$m bis 20 $\mu$m, Fa. ECKART GmbH) in 525 g Butylacetat 85/100 bei 200 rpm/min dispergiert und auf 40°C erhitzt. Anschließend wurde das gemäß Tabelle 6 verwendete Additiv in 30 g Butylacetat 85/100 zur Aluminiumeffektpigment-Dispersion zugegeben. Nach 6 Stunden rühren bei 90°C wurde die Mischung abgekühlt und über einen Büchnertrichter filtriert. Es wurden oberflächenmodifizierte Aluminiumeffektpigmente als 45-60%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung bei Verwendung der oberflächenmodifizierten Aluminiumeffektpigmente aus den Beispielen 26 bis 32 einen Nagellack mit spiegelähnlichem Glanz bzw. bei Verwendung des oberflächenmodifizierten Aluminiumeffektpigments aus Vergleichsbeispiel 1 einen Nagellack ohne spiegelähnlichen Glanz, mit matten Aluminium Grauton, ergaben.

Tabelle 6

| Beispiel/ Vergleichsbeispiel | Additiv | Menge [g] | NFA [%] |
|---|---|---|---|
| Beispiel 25 | Hostaphat CC 100 | 2,5 | 50 |
| Beispiel 26 | Hostaphat CC 100 | 5 | 53 |
| Beispiel 27 | Hostaphat CC 100 | 7,5 | 53 |
| Beispiel 28 | Hostaphat CC 100 | 20 | 61 |
| Beispiel 29 | Hostaphat CS 120 | 5 | 55 |
| Beispiel 30 | Hostaphat CS 120 | 10 | 53 |
| Beispiel 31 | Amphisol[7] | 10 | 46 |
| Vergleichsbeispiel 1 | Hostaphat CS 120 | 20 | 58 |
| [7] 1-Hexadecanolphosphat, 2,2'-Iminobis[ethanol] 1:1, CAS 69331-39-1, Fa. DSM. | | | |

Vergleichsbeispiele 2 bis 8:

In einem 1L-Doppelwandreaktor wurden 300 g des Aluminiumeffektpigments METALURE L 55350 AE (Dispersion in Essigsäureethylester, Feststoffgehalt 10%, $D_{50}$ (Horiba LA-930) = 11 $\mu$m bis 12 $\mu$m, Fa. ECKART GmbH) in 300 g Butylacetat 85/100 bei 200 rpm/min dispergiert und auf 100°C erhitzt. Anschließend wurde das gemäß Tabelle 7 verwendete Additiv in 30 g Butylacetat 85/100 zur Aluminiumeffektpigment-Dispersion zugegeben. Nach 6 Stunden rühren bei 40°C wurde die Mischung abgekühlt und über einen Büchnertrichter filtriert. Es wurden oberflächenmodifizierte Aluminiumeffektpigmente jeweils als 10-20%ige Dispersion erhalten, welche nach Einarbeitung in den Klarlack gemäß IIa (Pigmentierungshöhe: 0,4 Gew.-%, bezogen auf Gesamtgewicht des Klarlacks), Applikation auf einen künstlichen Fingernagel und anschließender Trocknung einen Nagellack ohne spiegelähnlichen Glanz, hingegen einen Nagellack mit matten Aluminium Grauton, ergaben.

Tabelle 7

| Vergleichsbeispiel | Additiv | Menge [g] | NFA [%] |
|---|---|---|---|
| 2 | SilCare Silicone 41 M80[8] | 3 | 18 |
| 3 | SilCare Silicone 41M80 | 15 | 15 |

(fortgesetzt)

| Vergleichsbeispiel | Additiv | Menge [g] | NFA [%] |
|---|---|---|---|
| 4 | Hostaphat KW 340 D[9) | 3 | 13 |
| 5 | Hostaphat KW 340 D | 15 | 13 |
| 6 | Hostaphat KL 340 D[10) | 3 | 16 |
| 7 | Hostaphat KL 340 D | 15 | 14 |
| [8) INCI: C24-28 Alkyl Dimethicone, CAS-Nummer: 192230-29-8, Fa. Aako BV oder Fa. Clariant. [9) Mono-, di- und tri-(alkyltetraglycolether)-o-phosphorsäurester, CAS-Nummer: 119415-05-3, Fa. Clariant. [10) Mono-, di- und tri-(alkyltetraglycolether)-o-phosphorsäureester, CAS-Nummer: 121158-63-2; 121158-61-0; 121158-62-1, Fa. Clariant. | | | |

Vergleichsbeispiele 9 bis 11:

In einem 1L-Doppelwandreaktor wurden 300 g Silverdream Moonlight 50IL (Feststoffgehalt 50%. $D_{50}$ (CILAS 1064) = 15 $\mu$m bis 20 $\mu$m, Fa. ECKART GmbH), in 470 g Butylacetat 85/100 bei 200 rpm/min dispergiert und auf 90°C erhitzt. Anschließend wurde das gemäß Tabelle 8 verwendete Additiv in 30 g Butylacetat 85/100 zur Aluminiumeffektpigment-Dispersion zugegeben. Nach 6 Stunden rühren bei 40°C wurde die Mischung abgekühlt und über einen Büchnertrichter filtriert. Es wurden oberflächenmodifizierte Aluminiumeffektpigmente als 55-65%ige Dispersion erhalten, welche nach Einarbeitung in den Klarlack gemäß IIa (Pigmentierungshöhe: 1,5 Gew.-%, bezogen auf Gesamtgewicht des Klarlacks), Applikation auf einen künstlichen Fingernagel und anschließender Trocknung einen Nagellack ohne spiegelähnlichen Glanz und/oder ohne Leafing-Effekt ergaben.

Tabelle 8

| Vergleichsbeispiel | Additiv | Menge [g] | NFA [%] |
|---|---|---|---|
| 8 | Hostaphat CK 100[11) | 7,5 | 59 |
| 9 | Hostaphat CK 100 | 15 | 61 |
| 10 | Hostaphat CK 100 | 30 | 59 |
| 11 | Hostaphat CC 100 | 75 | 57 |
| [11) Kaliumhexadecylhydrogenphosphat, CAS 19035-79-1, Hostaphat CK 100; Fa. Clariant. | | | |

Vergleichsbeispiel 12:

PVD-Aluminiumpigmentdispersion in Ethylacetat METALURE A-41010 AE, Fa. ECKART GmbH, NFA: 10%, $D_{50}$ = 9,50 $\mu$m bis 10,50 $\mu$m.

Vergleichsbeispiel 13:

PVD-Aluminiumpigmentdispersion in Ethylacetat METALURE L-55350 AE, Fa. ECKART GmbH, NFA: 10%, $D_{50}$ = 11,00 $\mu$m bis 12,00 $\mu$m.

Vergleichsbeispiel 14:

PVD-Aluminiumpigmentdispersion in Ethylacetat METALURE A-31017 AE, Fa. ECKART GmbH, NFA: 10%, $D_{50}$ = 9,50 bis 10,50 $\mu$m.

Vergleichsbeispiel 15:

Aluminiumpigmentpaste SILVERSHINE S2100, Fa. ECKART GmbH, NFA: 48,0% bis 52,0%, $D_{50}$ = 17,0 $\mu$m bis 23,0 $\mu$m.

Vergleichsbeispiel 16:

Mischung aus 13 Gew.-% METALURE A-41010 AE, Fa. ECKART GmbH und 0,2 Gew.-% Hostaphat CS 120, Fa. Clariant.

**II Herstellung der erfindungsgemäßen Nagellackkompositionen**

IIa Herstellung des Klarlacks:

In einem geeigneten Rührgefäß wurde eine 70 Gew.-%ige Bindemittellösung F100 hergestellt. Hierzu wurden 30 g Butylacetat 98/100 vorgelegt, unter Rühren und Kühlen (12 °C) mit dem Dissolver Dispermat CNf2 (Fa. Getzmann GmbH) 70 g des Bindemittels Kristalex F100 Hydrocarbon Resin (Fa. Eastman) zugegeben und anschließend 30 Minuten bei 3000 bis 4000 rpm/min nachgerührt.

In einem zweiten geeigneten Rührgefäß wurde eine 60 Gew.-%ige Bindemittellösung 5140 hergestellt, Hierzu wurden 40 g Butylacetat 98/100 vorgelegt, unter Rühren und Kühlen (12 °C) mit dem Dissolver Dispermat CNf2 (Fa. Getzmann GmbH) 60 g des Bindemittels Kristalex 5140 Hydrocarbon Resin (Fa. Eastman) zugegeben und anschließend 30 Minuten bei 3000 bis 4000 rpm/min nachgerührt.

Der nichtflüchtige Anteil (Bindemittelfestkörperanteil) der vorstehend beschriebenen Bindemittellösungen wurde gemäß DIN EN ISO 3251:2008 bestimmt.

Zur Herstellung des Klarlacks wurden die 70 Gew.-%ige Bindemittellösung F100 sowie die 60 Gew.-%ige Bindemittellösung 5140 gemäß nachstehender Tabelle unter Rühren mit 900 rpm/min mit dem Laborrührer IKA RW20 Digital (Fa. IKA) bei Raumtemperatur zusammengegeben und 5 bis 10 Minuten nachgerührt. Anschließend wurden die Lösungsmittel gemäß jeweiliger nachstehender Tabelle nacheinander unter Rühren mit 600 rpm/min zugegeben.

IIb Herstellung der erfindungsgemäßen Nagellackkompositionen

Zur Herstellung der erfindungsgemäßen Nagellackkompositionen der Beispiele 32 bis 35 wurden 4 g des jeweiligen oberflächenmodifizierten Effektpigments gemäß nachstehenden Tabellen 9, 10 und 11 vorgelegt und 96 g des Klarlacks unter Rühren mit 600 rpm/min zugegeben.

Tabelle 9: Die erfindungsgemäßen Nagellackkompositionen der Beispiele 32 bis 35 wiesen einen Bindemittelfestkörperanteil von 30 Gew.-%, bezogen auf das Gesamtgewicht des Klarlacks, auf.

| Beispiel | | Produktname | Einwaage (g) | Gewichtsverhältnis |
|---|---|---|---|---|
| Beispiel 32 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 5,0 | |
| | | Ethylacetat | 18,7 | |
| | | Butylacetat 98/100 | 28,0 | |
| | | Effektpigment gemäß Beispiel 5, NFA: 23% | 4,0 | |
| Beispiel 33 | Klarlack | Kristalex F100 Hydrocarbon Resin, 70 Gew.-% in Butylacetat | 14,8 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 34,6 | |
| | | Isopropanol | 4,5 | |
| | | Ethylacetat | 19,0 | |
| | | Butylacetat 98/100 | 27,5 | |
| | | Effektpigment gemäß Beispiel 5, NFA: 23% | 4,0 | |
| Beispiel 34 | Klarlack | Kristalex F100 Hydrocarbon Resin, 70 Gew.-% in Butylacetat | 8,9 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 41,6 | |
| | | Isopropanol | 4,5 | |
| | | Ethylacetat | 19,0 | |
| | | Butylacetat 98/100 | 26,0 | |
| | | Effektpigment gemäß Beispiel 5, NFA: 23% | 4,0 | |
| Beispiel 35 | Klarlack | Kristalex F100 Hydrocarbon Resin, 70 Gew.-% in Butylacetat | 4,8 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 46,3 | |
| | | Isopropanol | 4,5 | |
| | | Ethylacetat | 17,9 | |
| | | Butylacetat 98/100 | 26,5 | |
| | | Effektpigment gemäß Beispiel 5, NFA: 23 | 4,0 | |

Tabelle 10: Die erfindungsgemäßen Nagellackkompositionen der Beispiele 36 bis 39 wiesen einen Bindemittelfestkörperanteil von 40 Gew.-%, bezogen auf das Gesamtgewicht des Klarlacks, auf.

| Beispiel | | Produktname | Einwaage (g) | Gewichtsverhältnis |
|---|---|---|---|---|
| Beispiel 36 | Klarlack | Kristalex F100 Hydrocarbon Resin, 70 Gew.-% in Butylacetat | 27,9 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 32,5 | |
| | | Isopropanol | 3,0 | |
| | | Ethylacetat | 11,6 | |
| | | Butylacetat 98/100 | 25,0 | |
| | | Effektpigment gemäß Beispiel 5, NFA: 23% | 4,0 | |
| Beispiel 37 | Klarlack | Kristalex F100 Hydrocarbon Resin, 70 Gew.-% in Butylacetat | 19,8 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 46,3 | |
| | | Isopropanol | 3,1 | |
| | | Ethylacetat | 12,3 | |
| | | Butylacetat 98/100 | 18,5 | |
| | | Effektpigment gemäß Beispiel 5, NFA: 23% | 4,0 | |
| Beispiel 38 | Klarlack | Kristalex F100 Hydrocarbon Resin, 70 Gew.-% in Butylacetat | 11,8 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 55,4 | |
| | | Isopropanol | 2,9 | |
| | | Ethylacetat | 11,9 | |
| | | Butylacetat 98/100 | 20,0 | |
| | | Effektpigment gemäß Beispiel 5, NFA: 23% | 4,0 | |
| Beispiel 39 | Klarlack | Kristalex F100 Hydrocarbon Resin, 70 Gew.-% in Butylacetat | 6,7 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 61,5 | |
| | | Isopropanol | 2,9 | |
| | | Ethylacetat | 11,5 | |
| | | Butylacetat 98/100 | 17,4 | |
| | | Effektpigment gemäß Beispiel 5, NFA: 23% | 4,0 | |

Tabelle 11: Die erfindungsgemäßen Nagellackkompositionen der Beispiele 40 bis 43 wiesen einen Bindemittelfestkörperanteil von 60 Gew.-%, bezogen auf das Gesamtgewicht des Klarlacks, auf.

| Beispiel | | Produktname | Einwaage (g) | Gewichtsverhältnis |
|---|---|---|---|---|
| Beispiel 40 | Klarlack | Kristalex F100 Hydrocarbon Resin, 70 Gew.-% in Butylacetat | 42,9 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 50,0 | |
| | | Isopropanol | 0,6 | |
| | | Ethylacetat | 2,6 | |
| | | Butylacetat 98/100 | 3,9 | |
| | | Effektpigment gemäß Beispiel 5, NFA: 23% | 4,0 | |
| Beispiel 41 | Klarheit | Kristalex F100 Hydrocarbon Resin, 70 Gew.-% in Butylacetat | 28,6 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 66,7 | |
| | | Isopropanol | 0,4 | |
| | | Ethylacetat | 1,7 | |
| | | Butylacetat 98/100 | 2,6 | |
| | | Effektpigment gemäß Beispiel 5, NFA: 23% | 4,0 | |
| Beispiel 42 | Klarheit | Kristalex F100 Hydrocarbon Resin, 70 Gew.-% in Butylacetat | 17,1 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 80,0 | |
| | | Isopropanol | 0,2 | |
| | | Ethylacetat | 1,0 | |
| | | Butylacetat 98/100 | 1,7 | |
| | | Effektpigment gemäß Beispiel 5, NFA: 23% | 4,0 | |
| Beispiel 43 | Klarheit | Kristalex F100 Hydrocarbon Resin, 70 Gew.-% in Butylacetat | 9,6 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 88,8 | |
| | | Isopropanol | 0,1 | |
| | | Ethylacetat | 0,6 | |
| | | Butylacetat 98/100 | 0,9 | |
| | | Effektpigment gemäß Beispiel 5, NFA: 23% | 4,0 | |

Das optische Erscheinungsbild der erfindungsgemäßen Nagellackkompositionen der Beispiele 32 bis 43 wurde nach Applikation auf einem künstlichen Fingernagel und anschließender Trocknung durch das jeweils eingesetzte oberflächenmodifizierte Effektpigment aus Beispiel 5 bestimmt. Das jeweils eingesetzte oberflächenmodifizierte Effektpigment zeigte in dem gemäß IIa hergestellten Klarlack ein ausgeprägtes Leafing-Verhalten und teilweise einen ausgeprägten Spiegelglanz. Im Hinblick auf das Applikationsverhalten wurde festgestellt, dass sich die erfindungsgemäßen Nagellackkompositionen mit einem Bindemittelfestkörperanteil von 60 Gew.-%, bezogen auf das Gesamtgewicht des Klarlacks, aufgrund ihrer höheren Viskosität schlechter applizieren ließen als die erfindungsgemäßen Nagellackapplikationen mit einem Bindemittelfestkörperanteil von 30 Gew.-% oder 40 Gew.-%, jeweils bezogen auf das Gesamtgewicht des Klarlacks.

Für die erfindungsgemäßen Nagellackkompositionen der Beispiele 44 bis 48 erfolgte die Herstellung des Klarlacks wie vorstehend unter IIa beschrieben. Zur Herstellung der erfindungsgemäßen Nagellackkompositionen der Beispiele 44 bis 48 wurden 4 g des jeweiligen oberflächenmodifizierten Effektpigments gemäß nachstehender Tabelle 12 vorgelegt und 96 g des Klarlacks unter Rühren mit 600 rpm/min zugegeben.

Tabelle 12:

| Pigmentierung, Proben | | Produktname | Einwaage (g) | Gewichtsverhältnis |
|---|---|---|---|---|
| Beispiel 44 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 4,0 | |
| Beispiel 45 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 4,8 | |
| Beispiel 46 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |

| | | | | |
|---|---|---|---|---|
| | | Effektpigment gemäß Beispiel 5, NFA: 23% | 2,0 | |
| Beispiel 47 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 7,0 | |
| Beispiel 48 | Klarlack | Kristalex F100 Hydrocarbon Resin, 70 Gew.-% in Butylacetat | 22,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 26,0 | |
| | | Isopropanol | 4,7 | |
| | | Ethylacetat | 19,0 | |
| | | Butylacetat 98/100 | 28,0 | |
| | | Effektpigment gemäß Beispiel 5, NFA: 23% | 9,0 | |

Für die erfindungsgemäßen Nagellackkompositionen der Beispiele 49 bis 64 erfolgte die Herstellung des Klarlacks gemäß Tabelle 13 wie vorstehend unter IIa beschrieben.

Tabelle 13: Klarlack für die Beispiele 49 bis 64 und die Vergleichsbeispiele 24 bis 30

| | | Produktname | Einwaage (g) | Gewichtsverhältnis |
|---|---|---|---|---|
| Klarlack für die Beispiele 49 bis 64 und Vergleichsbeispiele 26 bis 33 | | Kristalex F100 Hydrocarbon Resin, 70 Gew.-% in Butylacetat | 19,8 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 46,3 | |
| | | Isopropanol | 3,1 | |
| | | Ethylacetat | 12,3 | |
| | | Butylacetat 98/100 | 18,5 | |

Zur Herstellung der erfindungsgemäßen Nagellackkompositionen der Beispiele 49 bis 64 wurde das jeweilige oberflächenmodifizierte Effektpigments gemäß nachstehender Tabelle 14 vorgelegt und der Klarlack aus Tabelle 13 gemäß Tabelle 14 unter Rühren mit 600 rpm/min zugegeben. Die erfindungsgemäßen Nagellackkompositionen der Beispiele 50 bis 66 wiesen einen Bindemittelfestkörperanteil von 40 Gew.-%, bezogen auf das Gesamtgewicht des Klarlacks, auf.

Tabelle 14: Einwaagen an Effektpigmentdispersion und Klarlack für die Beispiele 49 bis 64.

| Beispiel | Nagellackkomposition | Einwaage (g) |
|---|---|---|
| 49 | Effektpigment gemäß Beispiel 1, NFA: 10% | 13,20 |
| | Klarlack aus Tabelle 13 | 86,80 |
| 50 | Effektpigment gemäß Beispiel 15, NFA: 35% | 3,80 |
| | Klarlack aus Tabelle 13 | 96,20 |

(fortgesetzt)

| Beispiel | Nagellackkomposition | Einwaage (g) |
|---|---|---|
| 51 | Effektpigment gemäß Beispiel 14, NFA: 10% | 14,60 |
| | Klarlack aus Tabelle 13 | 85,40 |
| 52 | Effektpigment gemäß Beispiel 11, NFA: 14% | 9,65 |
| | Klarlack aus Tabelle 13 | 90,35 |
| 53 | Effektpigment gemäß Beispiel 12, NFA: 15% | 9,65 |
| | Klarlack aus Tabelle 13 | 90,35 |
| 54 | Effektpigment gemäß Beispiel 10; NFA: 18% | 7,65 |
| | Klarlack aus Tabelle 13 | 92,35 |
| 55 | Effektpigment gemäß Beispiel 21, NFA: 18,5% | 7,36 |
| | Klarlack aus Tabelle 13 | 92,64 |
| 56 | Effektpigment gemäß Beispiel 45, NFA: 11,7% | 11,64 |
| | Klarlack aus Tabelle 13 | 88,36 |
| 57 | Effektpigment gemäß Beispiel 17, NFA: 12% | 11,44 |
| | Klarlack aus Tabelle 13 | 88,56 |
| 58 | Effektpigment gemäß Beispiel 18, NFA: 12% | 11,44 |
| | Klarlack aus Tabelle 13 | 88,56 |
| 59 | Effektpigment gemäß Beispiel 5, NFA: 23% | 6,00 |
| | Klarlack aus Tabelle 13 | 94,00 |
| 60 | Effektpigment gemäß Beispiel 26, NFA: 53% | 2,60 |
| | Klarlack aus Tabelle 13 | 97,40 |
| 61 | Effektpigment gemäß Beispiel 28, NFA: 61% | 2,23 |
| | Klarlack aus Tabelle 13 | 97,70 |
| 62 | Effektpigment gemäß Beispiel 29, NFA: 55% | 2,47 |
| | Klarlack aus Tabelle 13 | 97,53 |
| 63 | Effektpigment gemäß Beispiel 25, NFA: 50% | 2,72 |
| | Klarlack aus Tabelle 13 | 97,28 |
| 64 | Effektpigment gemäß Beispiel 27, NFA: 53% | 2,57 |
| | Klarlack aus Tabelle 13 | 97,43 |

Vergleichsbeispiele

Für die Nagellackkompositionen der Vergleichsbeispiele 17 bis 20 erfolgte die Herstellung des Klarlacks wie vorstehend unter IIa beschrieben. Zur Herstellung der Nagellackkompositionen der Vergleichsbeispiele 18 bis 20 wurden 4 g des jeweiligen oberflächenmodifizierten Effektpigments gemäß nachstehender Tabelle 15 vorgelegt und 96 g des Klarlacks unter Rühren mit 600 rpm/min zugegeben.

Tabelle 15:

| Vergleichs- beispiel | | Produktname | Einwaage (g) | Gewichtsverhältnis |
|---|---|---|---|---|
| Vergleichs- beispiel 17 | Klarlack | Kristalex F100 Hydrocarbon Resin, 70 Gew.-% in Butylacetat | 22,25 | Kristalex F100 Hydrocarbon Resin: Kristalex 5140 Hydrocarbon Resin 1 : 1 |
| | | Kristalex 5140 Hydrocarbon Resin, 60 Gew.-% in Butylacetat | 26,0 | |
| | | Isopropanol | 14,31 | |
| | | Ethylacetat | - | |
| | | Butylacetat 98/100 | 37,44 | |
| | | Effektpigment gemäß Beispiel 5 | 4,0 | |
| Vergleichs- beispiel 18 | Klarlack | Kristalex F100 Hydrocarbon Resin, 70 Gew.-% in Butylacetat | 19,13 | Kristalex F100 Hydrocarbon Resin: Kristalex 5140 Hydrocarbon Resin 1 : 1 |
| | | Kristalex 5140 Hydrocarbon Resin, 60 Gew.-% in Butylacetat | 19,13 | |
| | | Isopropanol | 22,33 | |
| | | Ethylacetat | 39,42 | |
| | | Butylacetat 98/100 | - | |
| | | Effektpigment gemäß Beispiel 5 | 4,0 | |
| Vergleichs- beispiel 19 | Klarlack | Kristalex F100 Hydrocarbon Resin, 70 Gew.-% in Butylacetat | 14,87 | Kristalex F100 Hydrocarbon Resin: Kristalex 5140 Hydrocarbon Resin 1 : 2 |
| | | Kristalex 5140 Hydrocarbon Resin, 60 Gew.-% in Butylacetat | 34,63 | |
| | | Isopropanol | 18,5 | |
| | | Ethylacetat | 32,0 | |
| | | Butylacetat 98/100 | - | |
| | | Effektpigment gemäß Beispiel 5 | 4,0 | |
| Vergleichs- beispiel 20 | Klarlack | Kristalex F100 Hydrocarbon Resin, 70 Gew.-% in Butylacetat | 14,9 | Kristalex F100 Hydrocarbon Resin: Kristalex 5140 Hydrocarbon Resin 1 : 2 |
| | | Kristalex 5140 Hydrocarbon Resin, 60 Gew.-% in Butylacetat | 34,69 | |
| | | Isopropanol | 4,6 | |
| | | Ethylacetat | 15,18 | |
| | | Butylacetat 98/100 | 27,5 | |
| | | Aceton | 3,12 | |
| | | Effektpigment gemäß Beispiel 5 | 4,0 | |

Die Nagellackkompositionen der Vergleichsbeispiele 17 bis 20 zeigten nach der Applikation auf einem künstlichen Fingernagel und anschließender Trocknung ein deutlich schlechteres optisches Erscheinungsbild als die erfindungsgemäßen Nagellackkompositionen, was vermutlich auf das Weglassen eines der Lösungsmittel (Vergleichsbeispiel 17) und/oder einem zu hohen relativen Anteil der Lösemittel Isopropanol und/oder Ethylacetat im Verhältnis zu Butylacetat (Vergleichsbeispiele 18 und 19) zurückzuführen ist. Ferner wirkt sich das Hinzufügen eines zusätzlichen Lösungsmittels (Aceton in Vergleichsbeispiel 20) negativ aus. Die Nagellackapplikationen der Vergleichsbeispiele waren vermutlich aufgrund einer zu schnellen Trocknung weiß angelaufen und trüb. Die genauen Zusammensetzungen der Nagellacke der erfindungsgemäßen Beispiele und der Vergleichsbeispiele können auch Tabelle 18 entnommen werden.

Für die Nagellackkompositionen der Vergleichsbeispiele 21 bis 24 erfolgte die Herstellung des Klarlacks wie vorstehend unter IIa beschrieben. Zur Herstellung der Nagellackkompositionen der Vergleichsbeispiele 21 bis 24 wurden 4 g des jeweiligen oberflächenmodifizierten Effektpigments gemäß nachstehender Tabelle 16 vorgelegt und 96 g des Klarlacks unter Rühren mit 600 rpm/min zugegeben.

Die Nagellackkompositionen der Vergleichsbeispiele 21 bis 24 wiesen einen Bindemittelfestkörperanteil von 20 Gew.-%, bezogen auf das Gesamtgewicht des Klarlacks, auf.

Tabelle 16

| Vergleichs-beispiel | | Produktname | Einwaage (g) | Gewichtsverhältnis |
|---|---|---|---|---|
| Vergleichs-beispiel 21 | Klarlack | Kristalex F100 Hydrocarbon Resin, 70 Gew.-% in Butylacetat | 14,3 | Kristalex F100 Hydrocarbon Resin : Kristalex 5140 Hydrocarbon Resin 1 : 1 |
| | | Kristalex 5140 Hydrocarbon Resin, 60 Gew.-% in Butylacetat | 16,6 | |
| | | Isopropanol | 6,3 | |
| | | Ethylacetat | 25,1 | |
| | | Butylacetat 98/100 | 37,9 | |
| | | Effektpigment gemäß Beispiel 5, NFA: 23% | 4,0 | |
| Vergleichs-beispiel 22 | Klarlack | Kristalex F100 Hydrocarbon Resin, 70 Gew.-% in Butylacetat | 9,6 | Kristalex F100 Hydrocarbon Resin : Kristalex 5140 Hydrocarbon Resin 1 : 2 |
| | | Kristalex 5140 Hydrocarbon Resin, 60 Gew.-% in Butylacetat | 22,2 | |
| | | Isopropanol | 6,2 | |
| | | Ethylacetat | 24,8 | |
| | | Butylacetat 98/100 | 37,2 | |
| | | Effektpigment gemäß Beispiel 5, NFA: 23% | 4,0 | |
| Vergleichs-beispiel 23 | Klarlack | Kristalex F100 Hydrocarbon Resin, 70 Gew.-% in Butylacetat | 5,7 | Kristalex F100 Hydrocarbon Resin : Kristalex 5140 Hydrocarbon Resin 1 : 4 |
| | | Kristalex 5140 Hydrocarbon Resin, 60 Gew.-% in Butylacetat | 26,6 | |

| | | Isopropanol | 6,1 | |
|---|---|---|---|---|
| | | Ethylacetat | 24,4 | |
| | | Butylacetat 98/100 | 37,0 | |
| | | Effektpigment gemäß Beispiel 5, NFA: 23% | 4,0 | |
| Vergleichs-beispiel 24 | Klarlack | Kristalex F100 Hydrocarbon Resin, 70 Gew.-% in Butylacetat | 3,1 | Kristalex F100 Hydrocarbon Resin : Kristalex 5140 Hydrocarbon Resin 1 : 8 |
| | | Kristalex 5140 Hydrocarbon Resin, 60 Gew.-% in Butylacetat | 29,6 | |
| | | Isopropanol | 6,1 | |
| | | Ethylacetat | 24,5 | |
| | | Butylacetat 98/100 | 36,7 | |
| | | Effektpigment gemäß Beispiel 5, NFA: 23% | 4,0 | |

Die Nagellackkompositionen der Vergleichsbeispiele 21 bis 24 zeigten nach Applikation auf einem künstlichen Fingernagel und anschließender Trocknung ein deutlich schlechteres optisches Erscheinungsbild als die applizierten erfindungsgemäßen Nagellackapplikationen, was vermutlich auf den zu geringen Bindemittelfestkörperanteil von ca. 20 Gew.-%, bezogen auf das Gesamtgewicht des Klarlacks, zurückzuführen ist. Das oberflächenmodifizierte Effektpigment wurde vermutlich durch das im Klarlack vorhandene Lösungsmittel benetzt, weshalb sich das Effektpigment bei bzw. nach der Applikation nicht an der Oberfläche des Klarlacks orientieren konnte.

Zur Herstellung der Nagellackkompositionen der Vergleichsbeispiele 25 bis 30 wurde das jeweilige oberflächenmodifizierte Effektpigments gemäß nachstehender Tabelle 17 vorgelegt und der Klarlack aus Tabelle 13 gemäß Tabelle 17 unter Rühren mit 600 rpm/min zugegeben. Die Nagellackkompositionen der Vergleichsbeispiele 25 bis 30 wiesen einen Bindemittelfestkörperanteil von 40 Gew.-%, bezogen auf das Gesamtgewicht des Klarlacks, auf.

Tabelle 17:

| Vergleichs beispiel | Nagellackkomposition | Einwaage (g) |
|---|---|---|
| 25 | Effektpigment gemäß Vergleichsbeispiel 10, NFA: 59% | 2,30 |
| | Klarlack aus Tabelle 13 | 97,70 |
| 26 | Effektpigment gemäß Vergleichsbeispiel 1, NFA: 58% | 2,40 |
| | Klarlack aus Tabelle 13 | 97,60 |
| 27 | Effektpigment gemäß Vergleichsbeispiel 15, NFA: 48-52% | 2,70 |
| | Klarlack aus Tabelle 13 | 97,30 |
| 28 | Effektpigment gemäß Vergleichsbeispiel 12, NFA:10% | 13,00 |
| | Klarlack aus Tabelle 13 | 87,00 |
| 29 | Effektpigment gemäß Vergleichsbeispiel 13, NFA: 10% | 13,00 |
| | Klarlack aus Tabelle 13 | 87,00 |
| 30 | Effektpigment gemäß Vergleichsbeispiel 14, NFA: 10% | 13,00 |
| | Klarlack aus Tabelle 13 | 87,00 |

In Tabelle 18 sind die berechneten Kompositionen der erfindungsgemäßen Beispiele und der Vergleichsbeispiele, die sich mit der Variation der Nagellackparameter (bei immer demselben Effektpigment des Beispiels 5) befassen, zusammengefasst.

Tabelle 18:

| Probe | Kristallex F100 | Kristalex 5140 | Isopropanol | Ethylacetat | Butylacetat | weiteres Lösemittel | Pigment gem. Beispiel 5: | Summe | Gesamtgehalt BM in Gew.-% | Gehalt Effektpigment | Gehalt Iso | Gehalt Ethylacetat | Gehalt Butylacetat gesamt |
|---|---|---|---|---|---|---|---|---|---|---|---|---|---|
| Beispiel 32 | 22,3 | 26 | 5 | 18,7 | 28 | | 4 | 104 | 30,0 | 0,88 | 6,96 | 26,02 | 67,02 |
| Beispiel 33 | 14,8 | 34,6 | 4,5 | 19 | 27,5 | | 4 | 104,4 | 29,8 | 0,88 | 6,22 | 26,26 | 67,52 |
| Beispiel 34 | 8,9 | 41,6 | 4,5 | 19 | 26 | | 4 | 104 | 30,0 | 0,88 | 6,26 | 26,43 | 67,31 |
| Beispiel 35 | 4,8 | 46,3 | 4,5 | 17,9 | 26,5 | | 4 | 104 | 29,9 | 0,88 | 6,26 | 24,88 | 68,86 |
| Beispiel 36 | 27,9 | 32,5 | 3 | 11,6 | 25 | | 4 | 104 | 37,5 | 0,88 | 4,68 | 18,11 | 77,21 |
| Beispiel 37 | 19,8 | 46,3 | 3,1 | 12,3 | 18,5 | | 4 | 104 | 40,0 | 0,88 | 5,05 | 20,02 | 74,93 |
| Beispiel 38 | 11,8 | 55,4 | 2,9 | 11,9 | 20 | | 4 | 106 | 39,2 | 0,87 | 4,56 | 18,72 | 76,72 |
| Beispiel 39 | 6,7 | 61,5 | 2,9 | 11,5 | 17,4 | | 4 | 104 | 40,0 | 0,88 | 4,72 | 18,70 | 76,58 |
| Beispiel 40 | 42,9 | 50 | 0,6 | 2,6 | 3,9 | | 4 | 104 | 57,7 | 0,88 | 1,39 | 6,04 | 92,57 |
| Beispiel 41 | 28,6 | 66,7 | 0,4 | 1,7 | 2,6 | | 4 | 104 | 57,7 | 0,88 | 0,93 | 3,95 | 95,12 |
| Beispiel 42 | 17,1 | 80 | 0,2 | 1 | 1,7 | | 4 | 104 | 57,7 | 0,88 | 0,46 | 2,32 | 97,22 |
| Beispiel 43 | 9,6 | 88,8 | 0,1 | 0,6 | 0,9 | | 4 | 104 | 57,7 | 0,88 | 0,23 | 1,39 | 98,38 |
| Beispiel 44 | 22,3 | 26 | 4,7 | 19 | 28 | | 4 | 104 | 30,0 | 0,88 | 6,54 | 26,44 | 67,02 |
| Beispiel 45 | 22,3 | 26 | 4,7 | 19 | 28 | | 4,8 | 104,8 | 29,8 | 1,05 | 6,48 | 26,21 | 67,30 |
| Beispiel 46 | 22,3 | 26 | 4,7 | 19 | 28 | | 2 | 102 | 30,6 | 0,45 | 6,68 | 27,02 | 66,30 |
| Beispiel 47 | 22,3 | 26 | 4,7 | 19 | 28 | | 7 | 107 | 29,2 | 1,50 | 6,34 | 25,61 | 68,05 |
| Beispiel 48 | 22,3 | 26 | 4,7 | 19 | 28 | | 9 | 109 | 28,6 | 1,90 | 6,21 | 25,09 | 68,70 |
| Vergleichsbeispiel 17 | 22,25 | 26 | 14,31 | 0 | 37,44 | | 4 | 104 | 30,0 | 0,88 | 19,90 | 0,00 | 80,10 |
| Vergleichsbeispiel 18 | 19,13 | 19,13 | 22,33 | 39,42 | 0 | | 4 | 104,01 | 23,9 | 0,88 | 28,55 | 50,40 | 21,06 |
| Vergleichsbeispiel 19 | 14,87 | 23,63 | 18,5 | 32 | 0 | | 4 | 93 | 26,4 | 0,99 | 27,41 | 47,41 | 25,18 |

(fortgesetzt)

| Probe | Kristallex F100 | Kristalex 5140 | Isopropanol | Ethylacetat | Butylacetat | weiteres Lösemittel | Pigment gem. Beispiel 5: | Summe | Gesamtgehalt BM in Gew.-% | Gehalt Effektpigment | Gehalt Iso | Gehalt Ethylacetat | Gehalt Butylacetat gesamt |
|---|---|---|---|---|---|---|---|---|---|---|---|---|---|
| Vergleichsbeispiel 20 | 14,9 | 34,69 | 4,6 | 15,18 | 27,5 | 3,12 (Aceton) | 4 | 103,99 | 30,0 | 0,88 | 6,40 | 21,13 | 68,12 |
| Vergleichsbeispiel 21 | 14,3 | 16,6 | 6,3 | 25,1 | 37,9 | | 4 | 104,2 | 19,2 | 0,88 | 7,56 | 30,13 | 62,31 |
| Vergleichsbeispiel 22 | 9,6 | 22,2 | 6,2 | 24,8 | 37,2 | | 4 | 104 | 19,3 | 0,88 | 7,47 | 29,87 | 62,67 |
| Vergleichsbeispiel 23 | 5,7 | 26,6 | 6,1 | 24,4 | 37 | | 4 | 103,8 | 19,2 | 0,89 | 7,36 | 29,42 | 63,22 |
| Vergleichsbeispiel 24 | 3,1 | 29,6 | 6,1 | 24,5 | 36,7 | | 4 | 104 | 19,2 | 0,88 | 7,34 | 29,46 | 63,20 |

**III Charakterisierung der erfindungsgemäßen, bevorzugt in einer Nagellackkomposition zu verwendenden, oberflächenmodifizierten Effektpigmente sowie der oberflächenmodifizierten Effektpigmente**

IIIa Teilchengrößenmessung

Die Größenverteilungskurve der erfindungsgemäßen, bevorzugt in einer Nagellackkomposition zu verwendenden, oberflächenmodifizierten Effektpigmente, der oberflächenmodifizierten Effektpigmente gemäß Anspruch 1 sowie der Pigmente der Vergleichsbeispiele, jeweils basierend auf nichtmetallischen plättchenförmigen Substraten, wurde mit dem Gerät Mastersizer 2000, Fa. Malvern, gemäß Herstellerangaben bestimmt. Hierzu wurden ca. 0,1 g des jeweiligen Pigments als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die Mittelwerte gebildet. Die Auswertung der Streulichtsignale erfolgte dabei nach der Fraunhofer Methode.

Die Größenverteilungskurve der erfindungsgemäßen, bevorzugt in einer Nagellackkomposition zu verwendenden, oberflächenmodifizierten Effektpigmente, der oberflächenmodifizierten Effektpigmente gemäß Anspruch 1 und der Pigmente der Vergleichsbeispiele, jeweils basierend auf metallischen plättchenförmigen Substraten, wurde mit dem Gerät Cilas 1064, Fa. Quantachrome, bzw. dem Gerät Horiba LA-930, Fa. Horiba, jeweils gemäß Herstellerangaben vermessen. Hierzu wurden ca. 50 ml des jeweiligen Pigments in Isopropanol suspendiert, 300 Sekunden im Ultraschallbad (Gerät: Sonorex IK 52, Fa. Bandelin) behandelt und anschließend mittels einer Pasteurpipette in die Probenvorbereitungszelle des Messgerätes gegeben und mehrmals vermessen. Aus den einzelnen Messergebnissen wurden die Mittelwerte gebildet. Die Auswertung der Streulichtsignale erfolgte dabei nach der Fraunhofer Methode.

Unter der mittleren Teilchengröße $D_{50}$ wird im Rahmen dieser Erfindung der $D_{50}$-Wert der Summenhäufigkeitsverteilung der volumengemittelten Größenverteilungsfunktion, wie sie durch Laserbeugungsmethoden erhalten werden, verstanden. Der $D_{50}$-Wert gibt an, dass 50% der Pigmente einen volumengemittelten Durchmesser aufweisen, der gleich oder kleiner als der angegebene Wert, beispielsweise 20 $\mu$m, ist. Entsprechend gibt der $D_{10}$-bzw.$D_{90}$-Wert an, dass 10% bzw. 90%der Pigmente einen volumengemittelten Durchmesser aufweisen, der gleich oder kleiner als der jeweilige Messwert ist.

Der Span $\Delta$D, definiert als $\Delta D = \frac{D_{90} - D_{10}}{D_{50}},$ gibt die Breite der Teilchengrößenverteilung an. Im Hinblick auf das optische Erscheinungsbild der erfindungsgemäßen, bevorzugt in einer Nagellackkomposition zu verwendenden, oberflächenmodifizierten Effektpigmente bzw. der oberflächenmodifizierten Effektpigmente gemäß Anspruch 1 ist ein kleiner Wert von $\Delta$D, d.h. ein enger Span, bevorzugt.

**IIIb Bestimmung der mittleren Dicke der metallischen oder nichtmetallischen plättchenförmigen Substrate, Bestimmung der mittleren Gesamtdicke des oberflächenmodifizierten Effektpigments**

Die Bestimmung der mittleren Dicken $h_{50}$ der erfindungsgemäßen, bevorzugt in einer Nagellackkomposition zu verwendenden, oberflächenmodifizierten Effektpigmente, die oberflächenmodifizierten Effektpigmente gemäß Anspruch 1 sowie die Pigmente der Vergleichsbeispiele erfolgte gemäß der in der WO 2004/087816 A2 (Seiten 24 und 25) beschriebenen Methode mittels REM.

IIIc Bestimmung des Metalloxidgehaltes der synthetischen Glimmerplättchen

Die Metalloxidgehalte der synthetischen Glimmerplättchen wurden mittels Röntgenfluoreszenzanalyse (RFA) bestimmt. Hierzu wurden die synthetischen Glimmerplättchen in eine Lithiumtetraboratglastablette eingearbeitet, in Festprobenmessbechern fixiert und daraus vermessen. Als Messgerät diente das Gerät Advantix ARL, Fa. Thermo Scientific.

**IV Optische Charakterisierung der erfindungsgemäßen Nagellackkompositionen**

IVa Bestimmung von Glanz (20° Geometrie) und Haze

Zur objektiven Bestimmung des optischen Erscheinungsbilds der erfindungsgemäßen Nagellackkompositionen sowie der Nagellackkompositionen der Vergleichsbeispiele wurde die jeweilige Nagellackkomposition mittels einer Kastenrakel (Filmapplicator Erichsen System Wasag Model 288, Fa. Erichsen) in einer Nassfilmschichtdicke von 110 $\mu$m auf Glasplatten appliziert und anschließend bei Raumtemperatur getrocknet. Von den so applizierten Nagellackkompositionen wurde der Glanzwert (20° Geometrie) sowie der Hazewert (Hlog) mit dem Gerät haze gloss (Fa. BYK Gardner) vermessen.

Für die Glanzmessung wurde die 20° Geometrie für hochglänzende Oberflächen herangezogen (Byk-Gardner, Digitaler Katalog "Qualitätskontrolle für Lacke und Kunststoffe", Seite 16). Sowohl der Glanz (20° Geometrie) als auch der Haze (Hlog) wurde an mindestens 5 verschiedenen Stellen der Nagellackapplikation bestimmt. In nachstehender Tabelle 18 sind die hieraus gebildeten Mittelwerte für Glanz (20° Geometrie) und Haze (Hlog) aufgeführt.

**V Ergebnisse:**

In Tabelle 19 werden die Glanz- und Haze-Werte ausgewählter Beispiele und Vergleichsbeispiele aufgeführt. Ferner sind die Pigmentierungshöhen, die Bindemittelkonzentrationen und die Zusammensetzungen der Lösemittel in den Nagellackkompositionen berechnet worden.

Die in Tabelle 19 aufgeführten Glanz- und Haze-Werte sind nicht unabhängig voneinander zu betrachten. Somit kann eine Nagellackkomposition mit einem hohen Haze- sowie gleichzeitig einem niedrigen Glanzwert dennoch einen spiegelähnlichen Effekt aufweisen.

Die erfindungsgemäßen Nagellackkompositionen der Beispiele 48 bis 64 zeigten nach der Applikation ein ausgeprägtes leafing Verhalten bis hin zu einem spiegelähnlichen Effekt. Die optisch hochwertigsten Nagellackapplikationen mit hervorragend ausgebildetem Spiegeleffekt wurden dabei durch die Verwendung von PVD-Pigmenten beschichtet mit Phosphorsäurecetylester (Hostaphat CC 100) erhalten (Beispiele 49, 51, 54,56,57,58 und 59).

Die Nagellackkompositionen der Vergleichsbeispiele 27 bis 30, bei denen kein Additiv verwendet wurde, zeigten hingegen keinen leafing Effekt und zum Teil sogar eine starke Stippenbildung nach der Applikation auf. Dementsprechend schlecht fielen die Glanz- und Hazewerte aus.

Bei Vergleichsbeispiel 25 wurde das Kaliumsalz der Cetylphosphorsäureesters verwendet, was unvorteilhaft ist. Bei Vergleichsbeispiel 26 enthielt die Applikation Stippen. Vermutlich ist dies auf einen zu hohen Anteil des Additivs bei der Herstellung des beschichteten Metallpigments zurückzuführen.

Tabelle 19: Ergebnisse optischer Untersuchen und wichtigste Kompositionsparameter ausgewählter Beispiele und Vergleichsbeispiele

| Beispiel/ Vergleichs -beispiel | Effektpigmenttyp | Additiv | Additiv-Pigment-Verhältnts in % | Pigmentgehalt [Gew.-%] | Gehalt des bevorzugten 3-Lösemittelgemischs*** bzgl. dem gesamten Lösemittelgehalt | Gehalt Kohlenwasserstoffharz [Gew.-%] | Glanz (20° Geometrie) | Haze (Hlog) |
|---|---|---|---|---|---|---|---|---|
| Beispiel 49 | PVD* | CC 100 | 10 | 1,20 | 71,2 | 57,4 | 624,3 | 1353,9 |
| Beispiel 50 | Pt-$** | CC 100 | 4 | 1,28 | 95,8 | 63,6 | 137,5 | 1344,9 |
| Beispiel 51 | PVD | CC 100 | 10 | 1,33 | 95,8 | 63,6 | 358,4 | 1510,7 |
| Beispiel 52 | PVD | Laurylphosphon-säure | 13,3 | 1,19 | 75,9 | 56,4 | 84,1 | 1289,1 |
| Beispiel 53 | PVD | Laurylphosphon-säure | 13,3 | 1,28 | 78,7 | 59,7 | 130,7 | 1415,0 |
| Beispiel 54 | PVD | CC 100 | 20,0 | 1,15 | 90,8 | 59,7 | 221,2 | 1450,5 |
| Beispiel 55 | PVD | Laurylphosphon-säure | 20,0 | 1,13 | 83,3 | 61,0 | 120,2 | 1383,1 |
| Beispiel 56 | PVD | CC 100 | 10,0 | 1,24 | 84,8 | 61,2 | 149,6 | 1390,4 |
| Beispiel 57 | PVD | CC 100 | 20,0 | 1,14 | 88,5 | 58,4 | 240,1 | 1568,0 |
| Beispiel 58 | PVD | CC 100 | 40,0 | 0,98 | 88,4 | 58,5 | 455,8 | 1496,1 |
| Beispiel 59 | PVD | CC 100 | 10,0 | 1,25 | 88,4 | 58,5 | 176,6 | 1499,3 |
| Beispiel 60 | Pt-$ | CC 100 | 5,0 | 1,31 | 95,1 | 62,1 | 101,0 | 1290,0 |
| Beispiel 61 | Pt-$ | CC 100 | 20,0 | 1,13 | 100,0 | 64,4 | 107,0 | 1317,7 |
| Beispiel 62 | Pt-$ | CS 100 | 5,0 | 1,29 | 100,0 | 64,6 | 125,1 | 1366,0 |
| Beispiel 63 | Pt-$ | CC 100 | 2,5 | 1,33 | 100,0 | 64,5 | 121,1 | 1347,6 |
| Beispiel 64 | Pt-$ | CC 100 | 7,5 | 1,27 | 100,0 | 64,3 | 99,9 | 1289,0 |
| Vergleichsbeispiel 28 | PVD | -- | 0 | 1,30 | 100,0 | 36,2 | 101,8 | 121,6 |
| Vergleichsbeispiel 29 | PVD | -- | 0 | 1,30 | 100,0 | 36,2 | 19,2 | 565,6 |
| Vergleichsbeispiel 30 | PVD | -- | 0 | 1,30 | 100,0 | 36,2 | 100,4 | 134,5 |

EP 3 442 491 B1

37

(fortgesetzt)

| Beispiel/ Vergleichs-beispiel | Effektpigmenttyp | Additiv | Additiv-Pigment-Verhältnts in % | Pigmentgehalt [Gew.-%] | Gehalt des bevorzugten 3-Lösemittelgemischs*** bzgl. dem gesamten Lösemittelgehalt | Gehalt Kohlenwasserstoffharz [Gew.-%] | Glanz (20° Geometrie) | Haze (Hlog) |
|---|---|---|---|---|---|---|---|---|
| Vergleichsbeispiel 27 | Pt-$ | -- | 0 | 1,35 | 96,1 | 40,5 | 104,4 | 175,5 |
| Vergleichsbeispiel 25 | Pt-$ | Hostaphat CK 100 | 20 | 1,13 | 100,0 | 64,6 | 73,2 | 1154,9 |
| Vergleichsbeispiel 26 | Pt-$ | CS 120 | 20 | 1,16 | 100,0 | 64,5 | 125,2 | 1363,5 |

* Hier sind alle PVD-Pigmente lediglich mit "PVD" abgekürzt, die weiteren Details entnehme man den entsprechenden vorangegangenen Tabellen.

** Mit "Pt-$" werden in dieser Tabelle alle nassvermahlenen Pigmente mit einer mittleren Dicke von unter 100 nm bezeichnet. Weitere Details entnehme man den entsprechenden vorangegangenen Tabellen.

*** Mischung aus Essigsäureethylester, Butylacetat und Isopropanol.

**Patentansprüche**

1.  Nagellackkomposition enthaltend

    a) wenigstens ein mit einem Ausgangsmaterial (Additiv) oberflächenmodifiziertes Effektpigment wobei das Effektpigment ein plättchenförmiges Substrat und optional wenigstens eine auf dem Substrat aufgebrachte Beschichtung umfasst
    b) wenigstens ein Kohlenwasserstoffharz als Bindemittel und
    c) wenigstens ein Lösemittel oder Lösemittelgemisch,

    wobei als Ausgangsmaterial (Additiv) zur Oberflächenmodifizierung des Effektpigments wenigstens eine Verbindung aus der Gruppe bestehend aus phosphorsäureesterhaltigen, phosphonsäureesterhaltigen, phosphonsäurehaltigen, fettsäurehaltigen und/oder silanhaltigen Verbindungen oder Mischungen hiervon, entnommen wird.

2.  Nagellackkomposition nach Anspruch 1, wobei als Ausgangsmaterial (Additiv) Phosphorsäureestern und/oder Phosphorsäuren der allgemeinen Formel R-O-P(O)(OR')(OR") und/oder Phosphonsäureestern und/oder Phosphonsäuren der allgemeinen Formel
    R-P(O)(OR')(OR") handeln,
    wobei die Reste R, R' und R" folgende Bedeutung haben:
    R = linearer und/oder verzweigter Alkylrest mit einer Kohlenstoffkette aus einem Bereich von $C_{10}$ bis $C_{20}$ und R' = R" = H, linearer und/oder verzweigter Alkylrest mit einer Kohlenstoffkette aus einem Bereich von $C_1$ bis $C_6$, bevorzugt $C_1$ bis $C_3$, wobei R' und R" identisch oder verschieden voneinander sein können.

3.  Nagellackkomposition nach einem der Ansprüche 1 oder 2, enthaltend wenigstens ein oberflächenmodifiziertes Effektpigment umfassend ein optional mit wenigstens einem Metalloxid, Metallhydroxid und/oder Metalloxidhydrat beschichtetes, metallisches plättchenförmiges Substrat oder ein optional mit wenigstens einem Metalloxid, Metallhydroxid und/oder Metalloxidhydrat beschichtetes nichtmetallisches plättchenförmiges Substrat, wobei

    a) das metallische plättchenförmige Substrat durch Nassvermahlung hergestellt wird und einen $h_{50}$-Wert aus einem Bereich von 20 nm bis kleiner 100 nm aufweist und als Ausgangsmaterial (Additiv) zur Oberflächenmodifizierung

    i) Cetylphosphorsäureester oder Cetylphosphorsäure aus einem Bereich von 2 Gew.-% bis unter 40 Gew.-% oder
    ii) Phosphorsäurestearylester oder Stearylphosphat aus einem Bereich von 5 Gew.-% bis unter 20 Gew.-% oder
    iii) Phosphonsäuren der Formel R-P(O)(OH)$_2$ mit R = linearer Alkylrest mit einer Kohlenstoffkette aus einem Bereich von $C_8$ bis $C_{14}$, in einer Gesamtmenge bevorzugt aus einem Bereich von 4 Gew.-% bis 45 Gew.-% jeweils bezogen auf das Gesamtgewicht des optional beschichteten, metallischen plättchenförmigen Substrats verwendet wird oder

    b) das metallische plättchenförmige Substrat durch PVD-Verfahren hergestellt wird und eine mittlere Dicke $h_{50}$ aus einem Bereich von 13 nm bis 60 nm aufweist und als Ausgangsmaterial (Additiv) zur Oberflächenmodifizierung

    i) Cetylphosphorsäureester oder Cetylphosphorsäure aus einem Bereich von 10 Gew.-% bis 50 Gew.-% oder
    ii) Phosphorsäurestearylester oder Stearylphosphat aus einem Bereich von 13 Gew.-% bis 50 Gew.-% oder
    iii) Phosphonsäuren der Formel R-P(O)(OH)$_2$ mit R = linearer Alkylrest mit einer Kohlenstoffkette aus einem Bereich von $C_8$ bis $C_{14}$, in einer Gesamtmenge aus einem Bereich von 5 Gew.-% bis 50 Gew.-%,, jeweils bezogen auf das Gesamtgewicht des optional beschichteten, metallischen plättchenförmigen Substrats, verwendet wird oder

    c) das nichtmetallische plättchenförmige Substrat einen $D_{50}$-Wert aus einem Bereich von 2 $\mu$m bis 360 $\mu$m aufweist und als Ausgangsmaterial (Additiv) zur Oberflächenmodifizierung

    i) Cetylphosphorsäureester oder Cetylphosphorsäure aus einem Bereich von 5 Gew.-% bis 30 Gew.-% oder
    ii) Phosphorsäurestearylester oder Stearylphosphat aus einem Bereich von 2 Gew.-% bis 25 Gew.-%,

jeweils bezogen auf das Gesamtgewicht des optional beschichteten, nichtmetallischen plättchenförmigen Substrats, verwendet wird oder

d) das nichtmetallische Substrat ein Glasplättchen ist, welches mit metallischem Silber beschichtet ist und einen $D_{50}$-Wert aus einem Bereich von 2 $\mu$m bis 360 $\mu$m sowie eine mittlere Dicke $h_{50,Glas}$ des Glasplättchens in einem Bereich von 70 nm bis 530 nm sowie eine mittlere Dicke der metallischen Silberbeschichtung in einem Bereich von 9 nm bis 27 nm aufweist und als Ausgangsmaterial (Additiv) zur Oberflächenmodifizierung

i) Cetylphosphorsäureester oder Cetylphosphorsäure aus einem Bereich von 3 Gew.-% bis unter 40 Gew.-% oder

ii) Phosphorsäurestearylester oder Stearylphosphat aus einem Bereich von 5 Gew.-% bis unter 30 Gew.-% oder

iii) Phosphonsäuren $R\text{-}P(O)(OH)_2$ mit R = linearer Alkylrest mit einer Kohlenstoffkette aus einem Bereich von $C_8$ bis $C_{14}$ in einem Anteil aus einem Bereich von 15 Gew.-% bis 43 Gew.-%, jeweils bezogen auf das Gesamtgewicht des mit Silber beschichteten Glasplättchens, verwendet wird.

4. Nagellackkomposition nach Anspruch 3, enthaltend wenigstens ein oberflächenmodifiziertes Effektpigment, **dadurch gekennzeichnet;** **dass** das metallische plättchenförmige Substrat ein Aluminiumeffektpigment ist und nach

a) durch Nassvermahlung hergestellt wird und einen $D_{50}$-Wert aus einem Bereich von 8 $\mu$m bis 25 $\mu$m aufweist oder

nach b) durch PVD-Verfahren hergestellt wird und einen $D_{50}$-Wert aus einem Bereich von 2,5 $\mu$m bis 90 $\mu$m aufweist.

5. Nagellackkomposition nach einem der Ansprüche 1 bis 4, wobei als Ausgangsmaterial (Additiv) zur Oberflächenmodifizierung des Substrats oder der Beschichtung Laurylphosphonsäureester und insbesondere Laurylphosphonsäure eingesetzt wird.

6. Nagellackkomposition nach einem der Ansprüche 1 bis 5, wobei als Effektpigment ein PVD-Aluminiumpigment und als Ausgangsmaterial (Additiv) zur Oberflächenmodifizierung des Substrats oder der Beschichtung Cetylphosphorsäureester eingesetzt wird.

7. Nagellackkomposition nach einem der Ansprüche 1 bis 6, wobei das Kohlenwasserstoffharz 80 bis 100 Gew.-% des gesamten organischen Bindemittels ausmacht.

8. Nagellackkompositionen nach einem der Ansprüche 1 bis 7, wobei das Kohlenwasserstoffharz aus wenigstens zwei voneinander verschiedenen Kohlenwasserstoffharzen mit einem mittleren Molekulargewicht Mw von 1200 bis 1600 und einem mittleren Molekulargewicht Mw 4500 bis 5500 im Gewichtsverhältnis 1:1 bis 1:10 besteht.

9. Nagellackkompositionen nach einem der Ansprüche 1 bis 8, wobei der Gehalt an Kohlenwasserstoffharzen in einem Bereich von 25 Gew.-% bis 64 Gew.-%, bezogen auf die gesamte Nagellackkomposition, liegt.

10. Nagellackkomposition nach einem der Ansprüche 1 bis 9, enthaltend als Lösemittel eine Mischung aus Isopropanol, Ethylacetat und Butylacetat.

11. Nagellackkomposition nach Anspruch 10, wobei die Lösemittelmischung aus Isopropanol, Ethylacetat und Butylacetat 70 bis 100 Gew.-% des gesamten Lösemittels ausmacht.

12. Nagellackkomposition nach einem der Ansprüche 10 oder 11, wobei der Anteil von Isopropanol unter 20 Gew.-%, vorzugsweise unter 15 Gew.-%, bezogen auf das gesamte Lösemittel, liegt.

13. Nagellackkomposition nach einem der Ansprüche 1 bis 12 enthaltend zusätzlich Weichmacher, Antioxidantien, Antiabsetzmittel, Konservierungsstoffe, Öle, Wachse, Radikalfänger, Benetzungsadditive, Dispergierhilfsmittel, Benetzungshilfsmittel, Antischaummittel, Parfüm, Neutralisiermittel, Verdicker, UV-Blocker Feuchthaltemittel, Vitamine, Proteine und Mischungen hiervon.

14. Verfahren zur Herstellung der Nagellackkomposition nach den Ansprüchen 1 bis 13, umfassend die Schritte

i) Oberflächenmodifizierung des Effektpigments durch ein Additiv in einer Dispersion in einem Lösemittel,
ii) Lösen des Kohlenwasserstoffharz in einem Lösemittel oder Lösemittelgemisch
iii) Vermischen und Homogenisieren der Dispersion nach i) mit der Bindemittellösung nach ii).

**15.** Verfahren nach Anspruch 14, wobei Schritt i) gemäß einem Verfahren umfassend die folgenden Schritte:

i. Suspendieren des optional mit wenigstens einem Metalloxid, Metallhydroxid und/oder Metalloxidhydrat beschichteten, metallischen plättchenförmigen Substrats oder des optional mit wenigstens einem Metalloxid, Metallhydroxid und/oder Metalloxidhydrat beschichteten nichtmetallischen plättchenförmigen Substrats in wenigstens einem Lösemittel,
ii. Zugabe des Cetylphosphorsäureesters oder des Phosphorsäurestearylesters oder der Phosphonsäure bei optional erhöhter Temperatur zu der Suspension aus Schritt i. und Rühren der nun erhaltenen Suspension,
iii. Filtrieren, optional Trocknen, des gemäß Schritt ii. erhaltenen oberflächenmodifizierten Effektpigments,

erfolgt.

**16.** Verfahren zur Beschichtung eines natürlichen oder künstlichen Fingernagels umfassend die Schritte:

a) Beschichtung des natürlichen oder künstlichen Fingernagels mit einer Nagellackkomposition gemäß der Ansprüche 1 bis 13,
b) optional darauffolgende Beschichtung des Nagellacks mit einem Klarlack.

**17.** Verfahren nach Anspruch 16, **dadurch gekennzeichnet, dass** eine Beschichtung des Nagellacks mit einem lösemittelbasierendem Klarlack erfolgt, der auf Bindemitteln der Gruppe bestehend aus Polyvinyl Butyral, Polyvinylpyrrolidon und Mischungen hieraus, besteht.

**Claims**

1. Nail varnish composition comprising

a) at least one effect pigment surface-modified with a starting material (additive), where the effect pigment comprises a substrate in platelet form and optionally comprises at least one coating applied to the substrate,
b) at least one hydrocarbon resin as binder and
c) at least one solvent or solvent mixture,

wherein the starting material (additive) used for surface modification of the effect pigment is at least one compound from the group consisting of phosphoric ester-containing, phosphonic ester-containing, phosphonic acid-containing, fatty acid-containing and/or silane-containing compounds or mixtures thereof.

2. Nail varnish composition according to Claim 1, wherein as starting material (additive) phosphoric esters and/or phosphoric acids of the general formula R-O-P(O) (OR') (OR'') and/or phosphonic esters and/or phosphonic acids of the general formula R-P(O) (OR') (OR") are used,
where the R, R' and R" moieties are defined as follows:
R = linear and/or branched alkyl moiety having a carbon chain from a range from $C_{10}$ to $C_{20}$ and R' = R'' = H, linear and/or branched alkyl moiety having a carbon chain from a range from $C_1$ to $C_6$, preferably $C_1$ to $C_3$, wherein R' and R" may be identical or different.

3. Nail varnish composition according to either of Claims 1 and 2, comprising a surface-modified effect pigment comprising a metallic substrate in platelet form that is optionally coated with at least one metal oxide, metal hydroxide and/or metal oxide hydrate or a nonmetallic substrate in platelet form that is optionally coated with at least one metal oxide, metal hydroxide and/or metal oxide hydrate, wherein

a) the metallic substrate in platelet form is produced by wet grinding and has an hso from a range from 20 nm to less than 100 nm and the starting material (additive) used for surface modification is

i) cetylphosphoric ester or cetylphosphoric acid from a range from 2% by weight to less than 40% by weight or
ii) phosphoric acid stearyl ester or stearyl phosphate from a range from 5% by weight to less than 20% by weight or

iii) phosphonic acids of the formula $R\text{-}P(O)(OH)_2$ with R = linear alkyl radical having a carbon chain from a range from $C_8$ to $C_{14}$, in a total amount preferably from a range from 4% by weight to 45% by weight, based in each case on the total weight of the optionally coated metallic substrate in platelet form, or

b) the metallic substrate in platelet form is produced by PVD methods and has an average thickness $h_{50}$ from a range from 13 nm to 60 nm and the starting material (additive) used for surface modification is

i) cetylphosphoric ester or cetylphosphoric acid from a range from 10% by weight to 50% by weight or

ii) phosphoric acid stearyl ester or stearyl phosphate from a range from 13% by weight to 50% by weight or

iii) phosphonic acids of the formula $R\text{-}P(O)(OH)_2$ with R = linear alkyl radical having a carbon chain from a range from $C_8$ to $C_{14}$, in a total amount from a range from 5% by weight to 50% by weight, based in each case on the total weight of the optionally coated metallic substrate in platelet form, or

c) the nonmetallic substrate in platelet form has a $D_{50}$ from a range from 2 $\mu$m to 360 $\mu$m and the starting material (additive) used for surface modification is

i) cetylphosphoric ester or cetylphosphoric acid from a range from 5% by weight to 30% by weight or

ii) phosphoric acid stearyl ester or stearyl phosphate from a range from 2% by weight to 25% by weight, based in each case on the total weight of the optionally coated nonmetallic substrate in platelet form, or

d) the nonmetallic substrate is a glass platelet that has been coated with metallic silver and has a $D_{50}$ from a range from 2 $\mu$m to 360 $\mu$m and an average thickness $h_{50,glass}$ of the glass platelet within a range from 70 nm to 530 nm and an average thickness of the metallic silver coating in a range from 9 nm to 27 nm and the starting material (additive) used for surface modification is

i) cetylphosphoric ester or cetylphosphoric acid from a range from 3% by weight to less than 40% by weight or

ii) phosphoric acid stearyl ester or stearyl phosphate from a range from 5% by weight to less than 30% by weight or

iii) phosphonic acids $R\text{-}P(O)(OH)_2$ with R = linear alkyl moiety having a carbon chain from a range from $C_8$ to $C_{14}$, in a proportion from a range from 15% by weight to 43% by weight, based in each case on the total weight of the silver-coated glass platelet.

4. Nail varnish composition according to Claim 3, **characterized in that**
the metallic substrate in platelet form is an aluminum effect pigment and according to

a) is produced by wet grinding and has a $D_{50}$ value in a range from 8 $\mu$m to 25 $\mu$m or

according to b) is produced by PVD methods and has a $D_{50}$ in a range from 2.5 $\mu$m to 90 $\mu$m.

5. Nail varnish composition according to any of Claims 1 to 4, wherein the starting material (additive) used for surface modification of the substrate or the coating is laurylphosphonic ester and especially laurylphosphonic acid.

6. Nail varnish composition according to any of Claims 1 to 5, wherein the effect pigment used is a PVD aluminum pigment and the starting material (additive) used for surface modification of the substrate or the coating is phosphoric acid cetyl ester.

7. Nail varnish composition according to any of Claims 1 to 6, wherein the hydrocarbon resin makes up 80% to 100% by weight of the overall organic binder.

8. Nail varnish compositions according to any of Claims 1 to 7, wherein the hydrocarbon resin consists of at least two different hydrocarbon resins having an average molecular weight $M_w$ of 1200 to 1600 and an average molecular weight $M_w$ of 4500 to 5500 in a weight ratio of 1:1 to 1:10.

9. Nail varnish compositions according to any of Claims 1 to 8, wherein the content of hydrocarbon resins is within a range from 25% by weight to 64% by weight, based on the overall nail varnish composition.

10. Nail varnish composition according to any of Claims 1 to 9, comprising a mixture of isopropanol, ethyl acetate and butyl acetate as solvent.

11. Nail varnish composition according to Claim 10, wherein the solvent mixture of isopropanol, ethyl acetate and butyl acetate makes up 70% to 100% by weight of the overall solvent.

12. Nail varnish composition according to either of Claims 10 and 11, wherein the proportion of isopropanol is below 20% by weight, preferably below 15% by weight, based on the overall solvent.

13. Nail varnish composition according to any of Claims 1 to 12, additionally comprising plasticizers, antioxidants, antisettling agents, preservatives, oils, waxes, free-radical scavengers, wetting additives, dispersing aids, wetting agents, antifoams, perfume, neutralizing agents, thickeners, UV blockers, humectants, vitamins, proteins and mixtures thereof.

14. Process for producing the nail varnish composition according to Claims 1 to 13, comprising the steps of

    i) surface-modifying the effect pigment by means of an additive in a dispersion in a solvent,
    ii) dissolving the hydrocarbon resin in a solvent or solvent mixture,
    iii) mixing and homogenizing the dispersion according to i) with the binder solution according to ii).

15. Process according to Claim 14, wherein step i) is effected by a process comprising the following steps:

    i. suspending the metallic substrate in platelet form that has optionally been coated with at least one metal oxide, metal hydroxide and/or metal oxide hydrate or the nonmetallic substrate in platelet form that has optionally been coated with at least one metal oxide, metal hydroxide and/or metal oxide hydrate in at least one solvent,
    ii. adding the phosphoric acid cetyl ester or the phosphoric acid stearyl ester or the phosphonic acid at optionally elevated temperature to the suspension from step i. and stirring the suspension then obtained,
    iii. filtering, optionally drying, the surface-modified effect pigment obtained in step ii.

16. Method of coating a natural or synthetic fingernail, comprising the steps of:

    a) coating the natural or synthetic fingernail with a nail varnish composition according to Claims 1 to 13,
    b) optionally subsequently coating the nail varnish with a clearcoat.

17. Method according to Claim 16, **characterized in that** the nail varnish is coated with a solvent-based clearcoat consisting of binders from the group consisting of polyvinyl butyral, polyvinylpyrrolidone and mixtures thereof.

**Revendications**

1. Composition de vernis à ongles contenant :

    a) au moins un pigment à effet modifié en surface avec un matériau de départ (additif), le pigment à effet comprenant un substrat plaquettaire et éventuellement au moins un revêtement appliqué sur le substrat,
    b) au moins une résine hydrocarbonée en tant que liant et
    c) au moins un solvant ou mélange de solvants,

   en tant que matériau de départ (additif) pour la modification en surface du pigment à effet, au moins un composé étant extrait du groupe constitué par les composés contenant des esters d'acides phosphoriques, contenant des esters d'acides phosphoniques, contenant des acides phosphoniques, contenant des acides gras et/ou contenant des silanes ou des mélanges de ceux-ci.

2. Composition de vernis à ongles selon la revendication 1, dans laquelle, en tant que matériau de départ (additif), des esters d'acides phosphoriques et/ou des acides phosphoriques de la formule générale R-O-P(O)(OR')(OR'') et/ou des esters d'acides phosphoniques et/ou des acides phosphoniques de la formule générale R-P(O)(OR')(OR'') sont utilisés,
   les radicaux R, R' et R'' ayant la signification suivante :
   R = radical alkyle linéaire et/ou ramifié ayant une chaîne carbonée dans une plage allant de $C_{10}$ à $C_{20}$ et R' = R'' = H, radical alkyle linéaire et/ou ramifié ayant une chaîne carbonée dans une plage allant de $C_1$ à $C_6$, de préférence $C_1$ à $C_3$, R' et R'' pouvant être identiques ou différents l'un de l'autre.

3. Composition de vernis à ongles selon l'une quelconque des revendications 1 ou 2, contenant au moins un pigment à effet modifié en surface comprenant un substrat plaquettaire métallique, éventuellement revêtu avec au moins un oxyde de métal, un hydroxyde de métal et/ou un hydrate d'oxyde de métal, ou un substrat plaquettaire non métallique, éventuellement revêtu avec au moins un oxyde de métal, un hydroxyde de métal et/ou un hydrate d'oxyde de métal,

a) le substrat plaquettaire métallique étant fabriqué par broyage humide et présentant une valeur hso dans une plage allant de 20 nm à moins de 100 nm et, en tant que matériau de départ (additif) pour la modification en surface,

i) un ester d'acide cétylphosphorique ou de l'acide cétylphosphorique dans une plage allant de 2 % en poids à moins de 40 % en poids ou

ii) de l'ester stéarylique d'acide phosphorique ou du phosphate de stéaryle dans une plage allant de 5 % en poids à moins de 20 % en poids ou

iii) des acides phosphoniques de la formule $R-P(O)(OH)_2$ avec R = radical alkyle linéaire ayant une chaîne carbonée dans une plage allant de $C_8$ à $C_{14}$ en une quantité totale de préférence dans une plage allant de 4 % en poids à 45 % en poids, à chaque fois par rapport au poids total du substrat plaquettaire métallique, éventuellement revêtu, étant utilisés, ou

b) le substrat plaquettaire métallique étant fabriqué par des procédés de dépôt physique en phase vapeur et présentant une épaisseur moyenne $h_{50}$ dans une plage allant de 13 nm à 60 nm et, en tant que matériau de départ (additif) pour la modification en surface,

i) un ester d'acide cétylphosphorique ou de l'acide cétylphosphorique dans une plage allant de 10 % en poids à 50 % en poids ou

ii) de l'ester stéarylique d'acide phosphorique ou du phosphate de stéaryle dans une plage allant de 13 % en poids à 50 % en poids ou

iii) des acides phosphoniques de la formule $R-P(O)(OH)_2$ avec R = radical alkyle linéaire ayant une chaîne carbonée dans une plage allant de $C_8$ à $C_{14}$ en une quantité totale dans une plage allant de 5 % en poids à 50 % en poids, à chaque fois par rapport au poids total du substrat plaquettaire métallique éventuellement revêtu, étant utilisés, ou

c) le substrat plaquettaire non métallique présentant une valeur $D_{50}$ dans une plage allant de 2 $\mu$m à 360 $\mu$m et, en tant que matériau de départ (additif) pour la modification en surface,

i) un ester d'acide cétylphosphorique ou de l'acide cétylphosphorique dans une plage allant de 5 % en poids à 30 % en poids ou

ii) de l'ester stéarylique d'acide phosphorique ou du phosphate de stéaryle dans une plage allant de 2 % en poids à 25 % en poids,

à chaque fois par rapport au poids total du substrat plaquettaire non métallique éventuellement revêtu, étant utilisé, ou

d) le substrat non métallique étant une plaquette de verre, qui est revêtue avec de l'argent métallique et présente une valeur $D_{50}$ dans une plage allant de 2 $\mu$m à 360 $\mu$m, ainsi qu'une épaisseur moyenne hso,verre de la plaquette de verre dans une plage allant de 70 nm à 530 nm, ainsi qu'une épaisseur moyenne du revêtement d'argent métallique dans une plage allant de 9 nm à 27 nm et, en tant que matériau de départ (additif) pour la modification en surface,

i) un ester d'acide cétylphosphorique ou de l'acide cétylphosphorique dans une plage allant de 3 % en poids à moins de 40 % en poids, ou

ii) de l'ester stéarylique de l'acide phosphorique ou du phosphate de stéaryle dans une plage allant de 5 % en poids à moins de 30 % en poids ou

iii) des acides phosphoniques $R-P(O)(OH)_2$ avec R = radical alkyle linéaire ayant une chaîne carbonée dans une plage allant de $C_8$ à $C_{14}$ en une proportion dans une plage allant de 15 % en poids à 43 % en poids, à chaque fois par rapport au poids total de la plaquette de verre revêtue avec de l'argent, étant utilisés.

4. Composition de vernis à ongles selon la revendication 3, contenant au moins un pigment à effet modifié en surface, **caractérisée en ce que**

le substrat plaquettaire métallique est un pigment à effet d'aluminium et est fabriqué selon a) par broyage humide et présente une valeur $D_{50}$ dans une plage allant de 8 µm à 25 µm ou

est fabriqué selon b) par des procédés de dépôt physique en phase vapeur et présente une valeur $D_{50}$ dans une plage allant de 2,5 µm à 90 µm.

5. Composition de vernis à ongles selon l'une quelconque des revendications 1 à 4, dans laquelle, en tant que matériau de départ (additif) pour la modification en surface du substrat ou du revêtement, un ester d'acide laurylphosphonique et notamment de l'acide laurylphosphonique est utilisé.

6. Composition de vernis à ongles selon l'une quelconque des revendications 1 à 5, dans laquelle, en tant que pigment à effet, un pigment d'aluminium fabriqué par dépôt physique en phase vapeur est utilisé et, en tant que matériau de départ (additif) pour la modification en surface du substrat ou du revêtement, un ester d'acide cétylphosphorique est utilisé.

7. Composition de vernis à ongles selon l'une quelconque des revendications 1 à 6, dans laquelle la résine hydrocarbonée représente 80 à 100 % en poids du liant organique total.

8. Composition de vernis à ongles selon l'une quelconque des revendications 1 à 7, dans laquelle la résine hydrocarbonée est constituée par au moins deux résines hydrocarbonées différentes l'une de l'autre ayant un poids moléculaire moyen $M_w$ de 1 200 à 1 600 et un poids moléculaire moyen $M_w$ de 4 500 à 5 500 en un rapport en poids de 1:1 à 1:10.

9. Composition de vernis à ongles selon l'une quelconque des revendications 1 à 8, dans laquelle la teneur en résines hydrocarbonées se situe dans une plage allant de 25 % en poids à 64 % en poids, par rapport à la composition de vernis à ongles totale.

10. Composition de vernis à ongles selon l'une quelconque des revendications 1 à 9, contenant en tant que solvant un mélange d'isopropanol, d'acétate d'éthyle et d'acétate de butyle.

11. Composition de vernis à ongles selon la revendication 10, dans laquelle le mélange de solvants d'isopropanol, d'acétate d'éthyle et d'acétate de butyle représente 70 à 100 % en poids du solvant total.

12. Composition de vernis à ongles selon l'une quelconque des revendications 10 ou 11, dans laquelle la proportion d'isopropanol est inférieure à 20 % en poids, de préférence inférieure à 15 % en poids, par rapport au solvant total.

13. Composition de vernis à ongles selon l'une quelconque des revendications 1 à 12, contenant en outre des plastifiants, des antioxydants, des agents antidépôt, des conservateurs, des huiles, des cires, des capteurs de radicaux, des additifs de mouillage, des adjuvants de dispersion, des adjuvants de mouillage, des agents antimousse, des parfums, des agents de neutralisation, des épaississants, des agents bloquant les UV, des agents de rétention de l'humidité, des vitamines, des protéines et des mélanges de ceux-ci.

14. Procédé de fabrication de la composition de vernis à ongles selon les revendications 1 à 13, comprenant les étapes suivantes :

   i) la modification en surface du pigment à effet par un additif dans une dispersion dans un solvant,
   ii) la dissolution de la résine hydrocarbonée dans un solvant ou mélange de solvants,
   iii) le mélange et l'homogénéisation de la dispersion selon i) avec la solution de liant selon ii).

15. Procédé selon la revendication 14, dans lequel l'étape i) a lieu selon un procédé comprenant les étapes suivantes :

   i. la suspension du substrat plaquettaire métallique, éventuellement revêtu avec au moins un oxyde de métal, un hydroxyde de métal et/ou un hydrate d'oxyde de métal, ou du substrat plaquettaire non métallique, éventuellement revêtu avec au moins un oxyde de métal, un hydroxyde de métal et/ou un hydrate d'oxyde de métal, dans au moins un solvant,
   ii. l'ajout de l'ester d'acide cétylphosphorique ou de l'ester stéarylique d'acide phosphorique ou de l'acide phosphonique à température éventuellement élevée à la suspension de l'étape i. et l'agitation de la suspension alors obtenue,
   iii. la filtration, éventuellement le séchage, du pigment à effet modifié en surface obtenu selon l'étape ii.

**16.** Procédé de revêtement d'un ongle naturel ou artificiel comprenant les étapes suivantes :

   a) le revêtement de l'ongle naturel ou artificiel avec une composition de vernis à ongles selon les revendications 1 à 13,
   b) éventuellement le revêtement ultérieur du vernis à ongles avec un vernis transparent.

**17.** Procédé selon la revendication 16, **caractérisé en ce qu'**un revêtement du vernis à ongles avec un vernis transparent à base de solvant a lieu, qui est constitué de liants du groupe constitué par le polyvinylbutyral, la polyvinylpyrrolidone et des mélanges de ceux-ci.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1812518 A2
- EP 2227508 A1
- EP 2318463 A1
- EP 2576702 A1
- EP 1462085 A1
- EP 1299066 A2
- EP 1746913 A2
- EP 1792598 A1
- EP 1796794 A1
- EP 1082952 A1
- JP 2012081236 A
- EP 2248514 A2
- US 2010047199 A1
- DE 102007034928 A1
- EP 1251152 A1
- WO 9638505 A1
- WO 2005049739 A2
- WO 2010086165 A1

- WO 2004087816 A2
- WO 2008077612 A2
- WO 2009152941 A2
- EP 1980594 B1
- EP 1829833 B1
- EP 2042474 B1
- EP 289240 B1
- CN 102718229 A
- US 20140251184 A1
- EP 0723997 A1
- EP 2217664 A1
- EP 2346950 A1
- EP 2356181 A1
- EP 2346949 A1
- EP 2367889 A1
- WO 2007148758 A1
- WO 2010024283 A1
- EP 1796794 B2